(19) Europäisches Patentamt
European Patent Office
Office européen des brevets

(11) **EP 2 964 090 B1**

(12) **FASCICULE DE BREVET EUROPEEN**

(45) Date de publication et mention de la délivrance du brevet:
**23.11.2016 Bulletin 2016/47**

(21) Numéro de dépôt: **14710219.8**

(22) Date de dépôt: **06.03.2014**

(51) Int Cl.:
*B01L 3/00* (2006.01)      *A61B 5/15* (2006.01)
*A61B 5/151* (2006.01)      *A61B 5/157* (2006.01)
*A61B 10/00* (2006.01)      *G01N 1/10* (2006.01)

(86) Numéro de dépôt international:
**PCT/EP2014/054380**

(87) Numéro de publication internationale:
**WO 2014/135652 (12.09.2014 Gazette 2014/37)**

(54) **DISPOSITIF DE PRELEVEMENT D'UN ECHANTILLON DE LIQUIDE PAR CAPILLARITE ET PROCEDE D'ANALYSE ASSOCIE**

VORRICHTUNG ZUM GEWINNEN EINER FLÜSSIGEN PROBE DURCH KAPILLARWIRKUNG UND ZUGEHÖRIGES ANALYSEVERFAHREN

DEVICE FOR COLLECTING A LIQUID SAMPLE BY CAPILLARY ACTION AND RELATED ANALYSIS METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **07.03.2013  FR 1352050**

(43) Date de publication de la demande:
**13.01.2016  Bulletin 2016/02**

(73) Titulaire: **Commissariat à l'Énergie Atomique et aux Énergies Alternatives**
**75015 Paris (FR)**

(72) Inventeurs:
• **POUTEAU, Patrick**
  **F-38240 Meylan (FR)**

• **BERTHIER, Jean**
  **F-38240 Meylan (FR)**
• **POHER, Vincent**
  **F-62340 Guines (FR)**

(74) Mandataire: **Brevalex**
**95, rue d'Amsterdam**
**75378 Paris Cedex 8 (FR)**

(56) Documents cités:
**EP-A1- 0 821 784      EP-A1- 1 701 149**
**EP-A2- 0 388 170      WO-A1-01/72220**
**WO-A1-95/10357      FR-A1- 2 325 920**
**US-A- 3 363 503**

**Description**

**DOMAINE TECHNIQUE**

La présente invention se rapporte au domaine des dispositifs permettant la collecte d'échantillons de liquide, notamment des échantillons chimiques et/ou biologiques, destinés à être analysés. Elle concerne en particulier le domaine des dispositifs de prélèvement de liquide par capillarité.

L'invention propose ainsi un dispositif de prélèvement d'un échantillon de liquide par capillarité, ainsi qu'un procédé d'analyse d'un échantillon de liquide prélevé au moyen d'un tel dispositif.

**ÉTAT DE LA TECHNIQUE ANTÉRIEURE**

De nombreux dispositifs capables de permettre un prélèvement de liquide destiné à être analysé sont connus dans l'art antérieur.

Par exemple, on connait des dispositifs de test sous forme de bandelettes en papier fonctionnalisées avec des réactifs nécessaires à la réalisation de tests. L'échantillon de liquide est alors mis en contact à une extrémité de la bandelette et, sous l'effet de la porosité du papier et des forces capillaires, le liquide remplit progressivement la bandelette. Alors, un réactif présent sur la bandelette sous forme séchée ou lyophilisée se mélange avec le liquide au cours du remplissage et la réaction engendrée peut permettre l'analyse de l'échantillon de liquide.

Le brevet européen EP 0 821 784 B1 décrit encore un autre type de dispositif de collecte d'un échantillon de liquide destiné à être analysé. Ce dispositif comporte un microcanal fluidique pour l'écoulement du liquide présentant une force capillaire supérieure à celle d'une cuvette d'analyse située entre le microcanal fluidique et une cavité donnant sur l'extérieur du dispositif. Le microcanal fluidique débouche sur une première extrémité permettant la collecte de liquide et une deuxième extrémité ouverte sur l'extérieur.

Toutefois, la mise en oeuvre des solutions connues de l'art antérieur exposées précédemment, et notamment celle décrite dans le brevet européen EP 0 821 784 B1, soulève plusieurs contraintes.

Par exemple, le dispositif décrit dans le brevet européen EP 0 821 784 B1 présente, à l'usage, un inconvénient en termes de risque de contamination de l'extérieur du dispositif par le liquide prélevé. En effet, le microcanal fluidique est ouvert sur l'extérieur au niveau de ses deux extrémités et lorsque l'on introduit un liquide dans le dispositif, le liquide remplit le microcanal fluidique et la cuvette d'analyse, mais il peut s'écouler également vers l'extérieur, notamment au travers de la deuxième extrémité ouverte sur l'extérieur. Notamment, ceci peut se produire en raison de la force d'inertie s'appliquant sur le liquide remplissant le microcanal fluidique. De plus, le liquide n'est pas suffisamment maintenu à l'intérieur de la zone formée par le microcanal fluidique et la cuvette d'analyse, et il peut s'avérer que le liquide s'écoule également à l'extérieur du dispositif par l'intermédiaire de la cavité.

En outre, pour le cas où un dépôt de réactif dans le microcanal est requis, la configuration de ce dispositif n'est pas réellement adaptée pour obtenir un dépôt homogène du réactif dans le microcanal. Compte-tenu de l'étroitesse et de la profondeur du microcanal fluidique, il est en effet difficile de déposer ou de sécher de façon homogène un réactif.

Par ailleurs, la forme de la génératrice du microcanal, c'est-à-dire la forme de la direction selon laquelle s'étend le microcanal, de ce dispositif peut accentuer la formation de bulles d'air non souhaitables dans le dispositif lors du prélèvement de liquide.

Enfin, la configuration de la première extrémité du microcanal servant à collecter le liquide à analyser n'est pas satisfaisante pour permettre un rendement optimal du prélèvement et pour faciliter l'opération de collecte.

**EXPOSÉ DE L'INVENTION**

Il existe un besoin pour proposer une alternative aux dispositifs connus de l'art antérieur permettant de collecter un échantillon de liquide, en particulier chimique et/ou biologique, pour en effectuer l'analyse.

Il existe notamment un besoin pour concevoir un dispositif de prélèvement d'un tel échantillon de liquide qui puisse permettre un confinement du liquide pour éviter qu'au moins une partie du liquide prélevé ne s'échappe à l'extérieur du dispositif.

Il existe en outre un besoin pour permettre, si nécessaire, la réalisation d'un dépôt sensiblement homogène d'un réactif dans le dispositif, destiné à entrer en contact avec le liquide prélevé, pour parvenir notamment à de meilleurs résultats d'analyse, en particulier dans le cas d'analyses mesurées par signal optique où l'homogénéité du résultat est un critère de qualité.

Il existe également un besoin pour diminuer, voire empêcher, la formation de bulles d'air non souhaitées dans le dispositif lors du prélèvement d'un échantillon de liquide.

Enfin, il existe un besoin pour permettre d'améliorer et de faciliter l'ergonomie et le rendement du dispositif de prélèvement lors de la collecte de l'échantillon de liquide.

L'invention a pour but de remédier au moins partiellement aux besoins mentionnés ci-dessus et aux inconvénients relatifs aux réalisations de l'art antérieur.

L'invention a ainsi pour objet, selon l'un de ses aspects, un dispositif de prélèvement d'un échantillon de liquide par capillarité, comportant un canal d'écoulement du liquide délimité par deux parois internes du dispositif entre lesquelles s'étend un fond de canal, la distance séparant les deux parois internes diminuant en direction du fond de canal, le canal s'étendant entre une première extrémité de collecte, ouverte sur l'extérieur du dispositif et apte à recevoir le liquide, et une deuxième extrémité, de façon à permettre l'écoulement du liquide par capillarité le long du fond de canal depuis la première extrémité vers la deuxième extrémité.

Avantageusement, le canal comporte, au niveau de la deuxième extrémité, un moyen de blocage apte à bloquer l'écoulement de liquide dans le canal depuis la première extrémité vers la deuxième extrémité. Ce moyen de blocage permet de bloquer l'écoulement de liquide ou de ralentir.

Grâce à cette caractéristique de l'invention, il peut être possible de remplir le canal du dispositif avec l'échantillon de liquide prélevé par effet capillaire en diminuant fortement, voire en empêchant complètement, le risque de débordement de liquide à l'extérieur du dispositif grâce à la présence du moyen de blocage. On peut ainsi réaliser un confinement de l'échantillon de liquide dans le dispositif et éviter le risque de contamination du milieu extérieur par du liquide provenant du dispositif.

Le dispositif selon l'invention peut en outre comporter l'une ou plusieurs des caractéristiques suivantes prises isolément ou suivant toutes combinaisons techniques possibles.

Le dispositif peut être utilisé pour l'analyse d'échantillons de liquide (ou volumes de liquide) prélevés à l'aide de celui-ci. En particulier, le dispositif peut être utilisé dans le domaine du test de diagnostic in vitro, et notamment pour des tests biologiques réalisés à proximité d'un patient permettant ainsi de délocaliser l'analyse en dehors d'un laboratoire d'analyse et la réaliser proche du patient. A cette fin, le dispositif peut être prévu pour être un consommable jetable, après analyse au moyen d'une instrumentation de lecture appropriée du dispositif.

Le liquide à prélever pour analyse peut être de tout type, étant en particulier un liquide biologique, notamment un liquide corporel tel que du sang, de l'urine, de la sueur, du liquide lacrymal, du liquide lymphatique, du sperme, entre autres.

L'analyse du liquide peut être réalisée par des moyens d'analyse appropriés, notamment des moyens d'analyse optiques. Ces moyens d'analyse peuvent être disposés de part et d'autre du canal. Autrement dit, le dispositif peut par exemple être inséré dans un moyen d'analyse de façon à permettre une analyse du liquide contenu dans le canal.

L'analyse du liquide peut être également réalisée par des moyens d'analyse électriques. Ces moyens d'analyse peuvent notamment comporter des électrodes, intégrées par exemple sur une paroi interne.

Une zone d'analyse ou zone d'intérêt, notamment une zone de mesure optique, peut être définie sur le canal pour permettre l'analyse du liquide. Par exemple dans le cadre d'une analyse optique, la zone d'analyse peut être éclairée par un faisceau lumineux et un capteur optique peut générer une image de la zone analysée, le traitement de cette image renseignant sur au moins un paramètre du liquide analysé.

Le moyen de blocage permet de ralentir, voire d'arrêter, l'écoulement du liquide dans sa progression d'écoulement depuis la première extrémité vers la deuxième extrémité du canal.

Le moyen de blocage peut être de différent type. Par exemple, le canal peut comporter un moyen de blocage sous la forme d'une paroi de fermeture du canal. La paroi de fermeture peut notamment être située au niveau de la deuxième extrémité du canal, s'étendant notamment entre les deux parois internes du dispositif. La paroi de fermeture peut ainsi fermer le canal et bloquer tout écoulement du liquide au-delà de celle-ci.

Le canal peut encore comporter un moyen de blocage sous la forme d'au moins une arête de blocage formée sur au moins une paroi interne (ou paroi latérale) du dispositif permettant un élargissement d'au moins une partie du canal. Autrement dit, la largeur d'au moins une partie du canal peut augmenter en aval de l'arête de blocage en considérant l'écoulement du liquide depuis la première extrémité vers la deuxième extrémité. L'arête de blocage peut en particulier permettre un élargissement du fond du canal.

L'arête de blocage peut être située en amont de la deuxième extrémité en considérant l'écoulement du liquide depuis la première extrémité vers la deuxième extrémité.

De préférence, le canal peut comporter deux arêtes de blocage situées sur chaque paroi interne. Les deux arêtes de blocage peuvent être disposées sensiblement en vis-à-vis, c'est-à-dire situées sur les parois internes sensiblement à une même hauteur du canal.

L'élargissement du canal peut dépendre de l'angle formé par l'arête de blocage avec la paroi interne du dispositif en cas de non élargissement. Ainsi, en cas de non élargissement (non présence d'arête de blocage), cet angle est nul. En cas d'élargissement, l'arête de blocage peut former un angle supérieur à 20°, de préférence supérieur à 50°, voire 60°, voire encore de préférence compris entre 80° et 100°. Le liquide peut alors s'accrocher à cette arête de blocage, ce qui bloque l'avancée du liquide dans le canal.

Par ailleurs, le canal peut encore comporter un moyen de blocage sous la forme d'un revêtement rendu localement hydrophobe pour empêcher l'écoulement du liquide.

Le revêtement peut notamment être situé au niveau du fond de canal et/ou au niveau d'au moins une paroi interne, par exemple les deux parois internes.

Le canal peut encore comporter un moyen de blocage sous la forme d'un élargissement, notamment progressif, du fond de canal, apte à diminuer la force capillaire s'appliquant sur le liquide, lorsque ce dernier progresse vers la deuxième extrémité.

En outre, le canal peut se diviser en au moins une partie inférieure comportant le fond de canal et une partie supérieure de telle sorte que la partie inférieure se situe entre le fond de canal et la partie supérieure. Les parties inférieure et supérieure sont délimitées par les parois internes.

Les parties inférieure et supérieure communiquent entre elles.

La partie inférieure présente une force capillaire supérieure à celle de la partie supérieure de façon à permettre un écoulement capillaire spontané (ECS ou SCF pour « Spontaneous Capillary Flow » en anglais) du liquide le long du fond de canal depuis la première extrémité vers la deuxième extrémité.

La partie inférieure peut ainsi former un microcanal d'écoulement du liquide ou encore un doigt fluidique d'écoulement du liquide rejoignant le fond de canal puis progressant depuis la première extrémité vers la deuxième extrémité.

La largeur de la partie supérieure est avantageusement supérieure à celle de la partie inférieure. Autrement dit, la partie supérieure peut constituer un élargissement de la partie inférieure.

La séparation entre les parties inférieure et supérieure du canal peut être réalisée par l'intermédiaire de moyens d'ancrage du liquide sur au moins une paroi interne du dispositif permettant un blocage de l'écoulement de liquide depuis la partie inférieure vers la partie supérieure.

Les moyens d'ancrage permettent un confinement du liquide à l'intérieur de la partie inférieure du canal. Autrement dit, les moyens d'ancrage peuvent permettre de maintenir le liquide dans la partie inférieure en deçà d'une hauteur prédéterminée du canal.

Avantageusement, chaque paroi interne comporte des moyens d'ancrage du liquide. Les moyens d'ancrage de chaque paroi interne peuvent être disposés sensiblement en vis-à-vis. Autrement dit, les moyens d'ancrage de chaque paroi interne peuvent être situés sensiblement à une même hauteur du canal.

Les moyens d'ancrage peuvent comporter des arêtes d'ancrage. Les arêtes d'ancrage peuvent permettre de délimiter la zone étroite de la partie inférieure de la zone large de la partie supérieure.

Dans un mode de réalisation de la partie inférieure du canal dans lequel le fond de canal s'étend perpendiculairement entre les parois internes, l'écoulement capillaire spontané ECS dans la partie inférieure du canal peut être obtenu lorsque la relation suivante est réalisée :

$$d_1 / (d_1 + 2h_1) < \cos \theta$$

où $d_1$ est la largeur de la partie inférieure,
$h_1$ est la hauteur de la partie inférieure, et
$\theta$ est l'angle de mouillage du liquide sur le matériau constituant la paroi interne, c'est-à-dire l'angle formé par une goutte de liquide avec le matériau au niveau de sa ligne triple. Plus cet angle est élevé, plus le liquide est hydrophile.

Pour que l'écoulement capillaire spontané puisse se produire, il est en tout cas nécessaire que la partie inférieure soit suffisamment étroite. En particulier, la largeur $d_1$ de la partie inférieure peut être inférieure ou égale à 1 mm, voire 500 $\mu$m, de préférence comprise entre 30 $\mu$m et 500 $\mu$m, voire entre 100 $\mu$m et 200 $\mu$m, notamment lorsque l'échantillon de liquide est du sang.

La largeur de la partie supérieure $w_1$ (au-delà des moyens d'ancrage) peut être choisie de sorte à être suffisamment importante pour ne pas permettre un écoulement capillaire spontané ECS dans la partie supérieure. Ainsi, l'élargissement depuis la partie inférieure vers la partie supérieure peut être de préférence d'au moins un facteur 2, de préférence d'au moins un facteur 3.

L'élargissement depuis la partie inférieure du canal vers la partie supérieure du canal peut dépendre de l'angle formé par l'arête d'ancrage avec la paroi interne du dispositif en cas de non élargissement. Ainsi, en cas de non élargissement (non présence d'arête d'ancrage), cet angle est nul. En cas d'élargissement, l'arête d'ancrage peut former un angle supérieur à 20°, de préférence supérieur à 50°, voire 60°, voire encore de préférence compris entre 80° et 100°. Autrement dit, l'angle formé par l'arête d'ancrage correspond à l'angle formé par la paroi interne de part et d'autre de l'arête d'ancrage.

Les moyens d'ancrage peuvent encore comporter un revêtement rendu localement hydrophobe. Ce revêtement peut être placé à l'interface entre la partie inférieure et la partie supérieure. Dans ce cas, l'élargissement du canal depuis la partie inférieure vers la partie supérieure peut ne pas être nécessaire. Autrement dit, la largeur de la partie supérieure peut ne pas être supérieure à celle de la partie inférieure. Les parties inférieure et supérieure peuvent par exemple avoir

4

la même largeur. Néanmoins, il est préférable d'avoir un élargissement du canal depuis la partie inférieure vers la partie supérieure, et donc que la partie supérieure présente une largeur supérieure à celle de la partie inférieure.

Ainsi, la présence des moyens de blocage et d'ancrage, tels que décrits ci-dessus, permet de confiner le liquide respectivement selon une direction longitudinale (c'est-à-dire de la première extrémité vers la deuxième extrémité), et selon une direction transversale (c'est-à-dire de la partie inférieure vers la partie supérieure). On évite alors tout risque de contact entre le liquide et l'extérieur du canal au niveau de la surface supérieure du canal, c'est-à-dire la surface rejoignant les extrémités des parois internes opposées au fond de canal. Ainsi, seule la première extrémité du canal peut être au contact du milieu extérieur.

De la sorte, lorsque le dispositif est inséré dans un moyen d'analyse, celui-ci n'est pas contaminé par le liquide contenu dans le canal, à la différence des solutions de l'art antérieur présentées précédemment.

Par ailleurs, les parois internes peuvent être sécantes, notamment au niveau de la partie inférieure du canal, de sorte à former le fond de canal à leur intersection. L'écoulement capillaire du liquide est ainsi facilité. Dans ce cas, le fond de canal correspond à l'intersection entre les deux parois internes.

De préférence, les deux parois internes forment à leur intersection un angle aigu inférieur à 40°. L'angle aigu ainsi formé peut permettre un écoulement capillaire spontané ECS par « effet de pointe ».

Selon une variante de réalisation, les parois internes se rapprochent, sans être sécantes. Dans ce cas, le fond de canal correspond à la surface joignant les deux parois internes dans la partie inférieure, lorsque l'écartement entre ces dernières est minimal. Cette surface peut être matérialisée par une paroi, dite paroi de fond.

Le dispositif selon l'invention peut en outre être fonctionnalisé par un réactif, notamment chimique ou biologique, destiné à réagir avec l'échantillon de liquide.

En particulier, le canal peut comporter sur au moins une partie de celui-ci au moins un réactif, notamment chimique ou biologique, sous forme sèche et/ou lyophilisée.

Le réactif peut notamment être situé en amont d'une zone d'analyse du canal, en considérant l'écoulement de liquide depuis la première extrémité vers la deuxième extrémité du canal. De la sorte, le liquide peut se mélanger au réactif lors de l'écoulement avant d'atteindre la zone d'analyse du canal.

Le réactif peut être déposé contre au moins une paroi interne de la partie inférieure du canal, par un procédé de séchage ou de lyophilisation. Lorsque le réactif est déposé par séchage, le dépôt est surfacique. Lorsque le réactif est déposé par lyophilisation, le dépôt est volumique.

Pour réaliser un tel dépôt, le réactif peut être introduit en phase liquide dans le canal. Le solvant liquide utilisé est généralement de l'eau. Le séchage s'effectue généralement à température ambiante et à pression atmosphérique ou bien sous vide par évaporation de la phase liquide, alors que la lyophilisation s'effectue généralement à basse pression et à basse température par sublimation de la phase solide. Dans ce dernier cas, la phase liquide, comportant le réactif, se solidifie sous l'effet de la basse température, généralement inférieure à 10°C. Le réactif solide est alors soumis à de basses pressions favorisant le passage de l'état solide à l'état gazeux. La phase solide se sublime. Dans le cas d'une lyophilisation, le réactif sous forme lyophilisée est présent dans le volume du canal fluidique sous forme d'une structure poreuse apte à se dissoudre rapidement en présence d'eau. Cette structure poreuse étant elle-même avide d'eau, elle contribue à la force de pompage du liquide, et contribue donc à son écoulement dans la partie inférieure du canal.

Le fait d'avoir une surface supérieure du canal, c'est-à-dire la surface rejoignant les extrémités des parois internes opposées au fond de canal, qui soit ouverte permet de favoriser le séchage ou la lyophilisation par une optimisation de la surface d'évaporation ou de sublimation de l'eau. Au contraire, un canal qui présenterait une surface supérieure fermée et des ouvertures uniquement à ses extrémités nécessiterait un temps beaucoup plus important pour réaliser l'évaporation ou la sublimation de l'eau.

Il est souhaitable que le dispositif favorise un dépôt le plus homogène possible du réactif sur la ou les parois internes du dispositif.

Pour cela, la hauteur du canal, et notamment la hauteur $h_1$ de la partie inférieure du canal, séparant le fond de canal de la partie supérieure du canal, est avantageusement la plus faible possible. De plus, la hauteur du canal, et notamment la hauteur $h_1$ de la partie inférieure du canal, reste avantageusement sensiblement constante en s'étendant le long du fond de canal depuis la première extrémité vers la deuxième extrémité.

La hauteur $h_1$ de la partie inférieure du canal peut notamment être inférieure ou égale à 5 mm, de préférence à 2 mm, voire à 1 mm, par exemple à 0,7 mm.

La hauteur $h_1$ de la partie inférieure peut par exemple être comprise entre 500 $\mu$m et 3 mm.

Le fait d'avoir une hauteur du canal, et notamment une hauteur de la partie inférieure du canal, qui reste sensiblement constante permet de maintenir des conditions géométriques d'évaporation ou de sublimation du solvant liquide constantes en fonction de l'avancée dans le canal, c'est-à-dire en fonction de la distance entre un point du canal et la première extrémité du canal. Ainsi, la quantité surfacique de réactif déposée (masse surfacique dans le cas d'un séchage et masse volumique dans le cas d'une lyophilisation) peut être identique quelle que soit l'avancée dans le canal. Cela permet de maîtriser la concentration en réactif, par élément de surface ou de volume, entre la première extrémité du canal et le point d'avancée dans le canal. Au contraire, dans les solutions de l'art antérieur exposées précédemment, la hauteur du canal ne reste pas constante et fluctue avec des zones d'augmention de hauteur significatives.

Par ailleurs, le fait d'avoir une hauteur du canal, et notamment une hauteur de la partie inférieure du canal, qui soit la plus faible possible peut également favoriser la présence homogène du réactif, car cela minise l'écart de distance entre le fond de canal et la partie supérieure du canal, destinée à rester au contact de l'air ambiant. Une faible hauteur $h_1$, telle que précédemment définie, facilite l'évacuation du solvant. De plus, cela permet de minimiser la quantité d'échantillon de liquide introduite dans le canal.

Il est également souhaitable de pouvoir éviter, ou au moins de pouvoir limiter, la formation de bulles d'air lors du prélèvement de l'échantillon de liquide avec le dispositif selon l'invention.

A cette fin, le canal peut s'étendre selon une direction sensiblement rectiligne depuis la première extrémité vers la deuxième extrémité.

En variante, le fond de canal peut s'étendre selon une direction sensiblement concave depuis la première extrémité vers la deuxième extrémité.

Par ailleurs, il est aussi souhaitable de faciliter et d'optimiser la collecte de l'échantillon de liquide avec le dispositif selon l'invention.

L'ouverture du canal vers l'extérieur du dispositif pour la collecte de l'échantillon de liquide, au niveau de la première extrémité, peut ainsi être de forme sensiblement arrondie, de façon notamment à rapprocher le fond de canal de l'ouverture.

Le dispositif peut comporter, au niveau de la première extrémité du canal, une surface de contact permettant le prélèvement de l'échantillon de liquide s'étendant dans un plan sensiblement perpendiculaire à au moins une paroi interne du dispositif.

La surface de contact est avantageusement destinée à être placée à proximité de l'élément dont on souhaite prélever un échantillon de liquide. En particulier, cet élément peut être un élément corporel, par exemple une extrémité de doigt, une lèvre ou tout autre membre dont on souhaite prélever un liquide corporel. La surface de contact peut ainsi être configurée pour permettre l'apposition de celle-ci sur un tel élément corporel. La surface de contact peut se conformer à un tel élément.

La surface de contact peut comporter une ouverture d'écoulement prévue pour déboucher dans le canal, par exemple dans la partie supérieure ou la partie inférieure du canal, par exemple au niveau du fond de canal. Ainsi, au niveau de la première extrémité du canal, le liquide prélevé peut s'écouler directement depuis la surface de contact vers le canal par l'intermédiaire de cette ouverture d'écoulement. Cette ouverture d'écoulement peut avoir une forme identique à la section du canal.

La surface de contact peut être évasée, étant notamment convexe vers la première extrémité du canal, pour permettre à l'échantillon de liquide prélevé de s'acheminer vers l'ouverture d'écoulement et ainsi vers le canal. Elle peut également être structurée, de façon à favoriser l'écoulement du liquide vers l'ouverture d'écoulement, par exemple par des microsillons convergeant vers ladite ouverture.

La présence d'une telle surface de contact peut permettre d'augmenter le rendement de collecte, et également d'éviter que du liquide prélevé ne s'écoule à l'extérieur du dispositif, notamment sur ses surfaces externes. Seuls le canal et la surface de contact sont alors susceptibles d'être en contact avec le liquide prélevé. Par ailleurs, la surface de contact peut agir en tant que surface d'appui contre laquelle l'élément, notamment un élément corporel, peut être appliqué avec une certaine pression. La pression exercé sur l'élément peut faciliter l'éjection de liquide corporel issu de l'élément corporel.

De préférence, la surface de contact est configurée pour que la partie centrale de l'élément corporel n'entre pas en contact direct avec l'élément dont on souhaite prélever un échantillon de liquide. Seul le liquide à la surface de l'élément entre en contact avec l'ouverture d'écoulement. La partie centrale de la surface de contact peut comporter au moins en partie l'ouverture d'écoulement prévue pour déboucher dans le canal, laquelle peut notamment être placée en face d'une incision, par exemple une piqure, réalisée sur un élément corporel par lequel s'écoule le liquide à prélever. Ainsi, la pression sur l'élément n'est exercée qu'en périphérie de l'incision, et non sur l'incision elle-même. Il est ainsi possible d'obtenir une bonne évacuation du liquide, tout en permettant l'application d'une pression sur l'élément concerné.

La surface de contact peut être elle-même structurée pour favoriser l'émergence du liquide corporel issu de l'élément corporel. Elle peut alors comporter une surface d'appui, contre laquelle l'élément corporel est destiné à s'appuyer pour le prélèvement de l'échantillon de liquide. Une telle surface d'appui peut être annulaire, et de préférence centrée par rapport à l'ouverture d'écoulement. Il peut par exemple s'agir d'un tore d'épaisseur de quelques millimètres, par exemple comprise entre 1 et 5 mm, de diamètre compris entre 5 mm et 1,5 cm, et centré autour de l'ouverture d'écoulement. Lorsque l'élément corporel est un doigt, ayant subi une incision, l'appui du doigt contre la surface d'appui favorise l'afflux de sang vers ladite incision. La surface d'appui, préférentiellement annulaire, peut être circulaire ou elliptique, pour se conformer à l'élément corporel duquel le liquide corporel est extrait.

La surface supérieure du canal, c'est-à-dire la surface rejoignant les extrémités des parois internes opposées au fond de canal, est de préférence ouverte, donnant sur l'extérieur. Quoi qu'il en soit, il est préférable que la surface supérieure du canal comporte une ouverture, de préférence à proximité de la deuxième extrémité, cette ouverture agissant en tant qu'évent pour chasser l'air initialement contenu dans le canal.

La présence d'une ouverture sur la surface supérieure du canal, notamment lorsque la surface supérieure du canal est totalement ouverte, permet de réaliser plus aisément un traitement chimique de l'intérieur du canal, notamment de la surface intérieure du canal. Par exemple, un traitement chimique de surface, notamment par plasma, par exemple plasma 02, peut permettre de diminuer l'angle de mouillage de l'échantillon de liquide par rapport à une paroi interne du dispositif.

Par ailleurs, les parois internes du dispositif peuvent être réalisées en un matériau transparent ou translucide, notamment au visible ou proche infrarouge. De la sorte, il peut être possible de réaliser une analyse optique du liquide contenu dans le canal. Une mesure optique peut en particulier être réalisée selon un axe optique sensiblement perpendiculaire aux parois internes délimitant le canal.

Pour permettre une mesure optique correcte, autour de la zone d'analyse du canal notamment, les parois internes sont de préférence d'une bonne qualité de planéité et de faible rugosité pour permettre une bonne transmission des rayons optiques. Aussi, de préférence, les parois internes sont sensiblement parallèles entre elles, notamment au niveau de la partie inférieure du canal. Cela permet d'éclairer le liquide contenu dans le canal par un faisceau optique sensiblement perpendiculaire aux deux parois internes. Autrement dit, cela permet de définir une direction normale aux deux parois internes selon laquelle on peut disposer un faisceau lumineux et l'axe optique d'un moyen d'analyse, en particulier un photodétecteur.

Le dispositif, et notamment les parois internes, peuvent par exemple être réalisés par moulage ou par injection en un matériau plastique tel que du polycarbonate, polypropylène, polyéthylène, cyclo oléfine copolymère (COC), cyclo oléfine polymère (COP), entre autres.

Le dispositif peut de plus comporter un moyen de percement, notamment une aiguille, pour permettre le perçage de la peau d'un patient afin de collecter l'échantillon de liquide. Ce moyen de percement peut préférentiellement être situé à proximité de la première extrémité du canal.

Ce moyen de percement peut être mobile dans le dispositif afin de réaliser l'étape d'incision ou de percement de la peau lorsque l'élément corporel est posé en appui sur la surface d'appui. Le moyen de percement mobile dans le dispositif peut être déployé, de façon à être appliqué rapidement contre l'élément corporel, de façon à former une incision dans ce dernier. Puis, il peut être replié dans le dispositif. Ainsi, le moyen de percement peut être déployé, d'une position de repli vers une position déployée, de façon à pratiquer l'incision, puis être replié dans ladite position de repli. Des moyens de rappel élastique peuvent permettre le déplacement du moyen de percement.

Dans le cas d'un moyen de percement fixe, par exemple une aiguille fixe, le dispositif muni d'un tel moyen de percement peut d'abord être utilisé dans un instrument de projection permettant au moyen de percement de frapper la peau du patient avec une vitesse pour la projection et une course pour la profondeur de perçage nécessaires pour réaliser une incision suffisante pour faire sortir une goutte d'échantillon de liquide, par exemple du sang, à la surface de la peau, puis réaliser le retrait du moyen de percement à une vitesse maîtrisée. Puis, le dispositif peut être utilisé pour prélever l'échantillon de liquide ainsi obtenu, par le biais de la première extrémité du canal.

Le dispositif peut comporter au moins deux canaux pour le prélèvement d'un même liquide ou d'au moins deux liquides distincts. Cela permet par exemple de comparer des mesures réalisées dans chaque canal. Les premières extrémités desdits au moins deux canaux peuvent être situées à proximité les unes des autres pour permettre notamment un contact simultané avec le ou les échantillons de liquide à prélever, par exemple une goutte de sang. Chaque canal d'un tel dispositif peut, le cas échéant, être rempli avec un réactif spécifique, à chaque fois différent, pour réaliser par exemple des tests de diagnostic multiparamétriques.

La hauteur du canal du dispositif peut être variable, augmentant au moins sur une première portion du canal.

En particulier, selon un mode de réalisation de l'invention, le canal peut comporter une partie concave, s'étendant sur une première portion comprise entre une première distance et une deuxième distance par rapport à la première extrémité du canal. Avantageusement, cette partie concave peut permettre une augmentation de la profondeur du canal.

De préférence, la première portion est située à proximité de la première extrémité. Une telle configuration peut permettre un drainage du liquide vers le fond de canal. Elle permet également un écoulement fluidique limitant le risque d'apparition de bulles d'air dans le canal.

L'invention a encore pour objet, selon un autre de ses aspects, un procédé d'analyse d'un échantillon de liquide prélevé au moyen d'un dispositif tel que défini précédemment, dans lequel on soumet le dispositif à des moyens d'analyse, aptes à analyser le liquide contenu dans le canal au niveau d'au moins une zone d'analyse prédéterminée du canal.

La zone d'analyse peut se situer à un niveau quelconque du canal. Par exemple, la zone d'analyse peut être située au centre du canal.

Le dispositif peut par exemple être introduit dans un moyen d'analyse optique de façon à permettre une analyse du liquide contenu dans la zone d'analyse du canal. La zone d'analyse peut être éclairée par un faisceau lumineux et un capteur optique peut générer une image de la zone analysée. Le traitement de cette image peut renseigner sur au moins un paramètre du liquide analysé.

Le procédé selon l'invention peut comporter l'une quelconque des caractéristiques précédemment énoncées, prises isolément ou selon toutes combinaisons techniquement possibles avec d'autres caractéristiques.

L'invention a encore pour objet, selon un autre de ses aspects, l'utilisation d'un dispositif tel que défini précédemment, pour le prélèvement d'un échantillon de liquide par capillarité, notamment une goutte de sang.

L'utilisation du dispositif pour le prélèvement de l'échantillon de liquide peut être effectuée alors qu'au moins un réactif est déjà présent dans le dispositif, notamment sur au moins une des parois internes pour un réactif séché ou en volume au niveau des parois internes pour un réactif lyophilisé.

De plus, avant son utilisation pour le prélèvement de l'échantillon de liquide, le dispositif peut être déjà présent dans des moyens d'analyse, notamment des moyens d'analyse optiques ou électriques.

L'échantillon de liquide, notamment la goutte de sang, peut être déjà présent sur l'élément corporel, notamment une extrémité de doigt, avant utilisation du dispositif. Ainsi, le dispositif peut par exemple être utilisé en le plaçant en appui sur l'élément corporel, par exemple à l'aide d'une surface d'appui du dispositif, pour permettre le prélèvement de l'échantillon de liquide.

En variante, l'échantillon de liquide, notamment la goutte de sang, n'est pas présent sur l'élément corporel, notamment une extrémité de doigt, avant utilisation du dispositif. Les moyens d'analyse peuvent alors comporter un moyen de percement, notamment une aiguille, pour permettre le perçage de la peau afin de collecter l'échantillon de liquide.

BRÈVE DESCRIPTION DES DESSINS

L'invention pourra être mieux comprise à la lecture de la description détaillée qui va suivre, d'exemples de mise en oeuvre non limitatifs de celle-ci, ainsi qu'à l'examen des figures, schématiques et partielles, du dessin annexé, sur lequel :

- la figure 1A représente un premier exemple d'un dispositif de prélèvement conforme à l'invention,
- la figure 1B représente une section selon B-B du dispositif de la figure 1A,
- la figure 1C est une vue de face selon C du dispositif de la figure 1A,
- les figures 2A, 2B et 2C illustrent les étapes de remplissage en liquide du dispositif de prélèvement de la figure 1A,
- les figures 3A et 3B illustrent deux exemples de réalisation de la partie inférieure du canal d'un dispositif conforme à l'invention,
- les figures 4A et 4B représentent la modélisation de l'écoulement de liquide dans le temps avec comparaison entre les comportements des parties inférieures des figures 3A et 3B,
- la figure 5A représente, en perspective, un deuxième exemple de réalisation d'un dispositif de prélèvement conforme à l'invention,
- la figure 5B est une vue agrandie du dispositif de prélèvement de la figure 5A,
- les figures 6A et 6B représentent, en perspective, un troisième exemple de réalisation d'un dispositif de prélèvement conforme à l'invention,
- la figure 7 représente un exemple de réalisation d'un canal d'un dispositif conforme à l'invention,
- la figure 8 illustre l'écoulement capillaire spontané ECS par effet de pointe dans le canal d'un dispositif conforme à l'invention,
- la figure 9 est un graphique représentant l'évolution de la hauteur maximale $h_{2max}$ de la partie supérieure du canal de la figure 7 en fonction de l'angle d'inclinaison ß des parois internes dans la partie supérieure, pour trois valeurs différentes de la largeur $w_1$ de la partie supérieure,
- les figures 10 et 11 représentent d'autres exemples de réalisation pour un canal d'un dispositif conforme à l'invention,
- les figures 12A, 12B, 12C et 12D illustrent des possibilités d'orientation de la direction selon laquelle s'étend un canal d'un dispositif conforme à l'invention,
- les figures 13A et 13B représentent un autre exemple de réalisation d'un dispositif conforme à l'invention comportant une surface d'appui et un moyen de percement, respectivement avec le moyen de percement en position déployée et en position de repli,
- la figure 14 représente la structure interne du dispositif des figures 13A et 13B,
- la figure 15 illustre une variante de réalisation du moyen de percement du dispositif des figures 13A et 13B,
- la figure 16 représente un autre exemple de réalisation d'un dispositif conforme à l'invention, comportant une surface d'appui,
- la figure 17A représente un autre exemple de réalisation d'un dispositif de prélèvement conforme à l'invention, avec une hauteur variable de canal,
- la figure 17B est une vue partielle en coupe selon B-B du dispositif de la figure 17A, et
- les figures 18A à 18D représentent une modélisation de l'écoulement d'un liquide au cours du temps, respectivement au cours de quatre étapes, dans le canal du dispositif de la figure 17A.

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

## EXPOSÉ DÉTAILLÉ DE MODES DE RÉALISATION PARTICULIERS

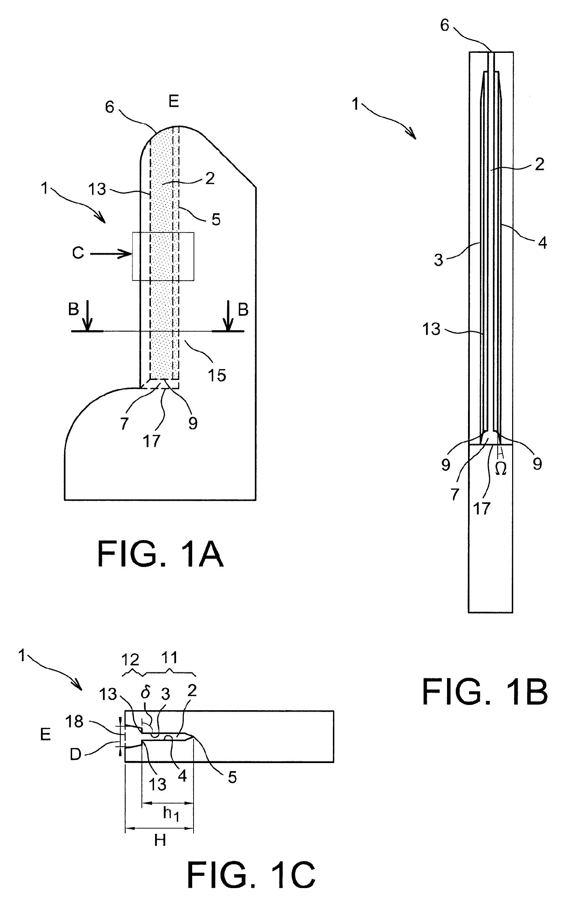

On a représenté sur les figures 1A, 1B et 1C un premier exemple de dispositif 1 de prélèvement d'un échantillon de liquide L par capillarité conforme à l'invention.

La figure 1A est une vue de profil du dispositif 1, la figure 1B est une vue en coupe selon B-B de la figure 1A et la figure 1C est une vue de face selon C du dispositif 1 de la figure 1A.

Conformément à l'invention, le dispositif 1 comporte un canal 2 d'écoulement du liquide L délimité par deux parois internes 3 et 4, ou parois latérales, entre lesquelles s'étend un fond 5 de canal. La distance D, visible sur la figure 1C, entre les deux parois internes 3 et 4 diminue en direction du fond 5 de canal. De plus, le canal 2 s'étend d'une première extrémité 6 de collecte, ouverte sur l'extérieur E du dispositif 1 et apte à recevoir le liquide L, et une deuxième extrémité 7, de façon à permettre l'écoulement du liquide L par capillarité le long du fond 5 de canal depuis la première extrémité 6 vers la deuxième extrémité 7.

Ainsi, la forme globale du canal 2, définie par les parois internes 3 et 4, va en s'amincissant depuis la surface supérieure 18 du canal 2, c'est-à-dire la surface rejoignant les extrémités des parois internes 3 et 4 opposées au fond 5 de canal, vers le fond 5 de canal, la surface supérieure 18 du canal 2 étant ouverte sur l'extérieur E. La forme du canal 2 allant en s'amincissant depuis cette surface supérieure 18 vers le fond 5 de canal permet d'obtenir une force de capillarité du canal 2 plus importante au niveau du fond 5 de canal qu'au niveau de la surface supérieure 18 ouverte sur l'extérieur E.

Par ailleurs, afin de maîtriser le remplissage du canal 2 en liquide L et éviter une éventuelle contamination de l'extérieur E par le liquide L, un ou plusieurs moyens de blocage de l'écoulement de liquide L le long du fond 5 de canal peuvent être prévus. Ces moyens permettent de bloquer l'écoulement de liquide au niveau de la deuxième extrémité ou en amont de cette dernière.

Un tel moyen de blocage peut par exemple se présenter sous la forme d'une paroi de fermeture 17 du canal 2, située notamment au niveau de la deuxième extrémité 7 du canal 2. Une telle paroi de fermeture 17 peut s'étendre transversalement entre les parois internes 3 et 4 pour fermer le canal 2, notamment au niveau de la deuxième extrémité 7.

Un tel moyen de blocage peut également se présenter sous la forme d'un revêtement rendu localement hydrophobe (non représenté) pour empêcher l'écoulement du liquide L. Ce revêtement peut être situé notamment au niveau de la deuxième extrémité 7 du canal 2 et par exemple au niveau du fond 5 de canal et/ou au niveau d'une ou des deux parois internes 3 et 4.

Dans l'exemple représenté des figures 1A, 1B et 1C, le canal 2 comporte deux moyens de blocage, un sur chaque paroi interne 3, 4, se présentant sous la forme de deux arêtes de blocage 9 qui permettent un élargissement d'une partie du canal 2, en particulier un élargissement de la deuxième extrémité 7 du canal 2. Les deux arêtes de blocage 9 sont en particulier visibles sur les figures 1A et 1C.

Les arêtes de blocage 9 peuvent permettre de stopper le remplissage capillaire du liquide L dans le canal 2 et peuvent ainsi permettre de maîtriser parfaitement le volume d'échantillon de liquide L prélevé par le dispositif 1.

Un moyen de blocage peut également comporter un élargissement progressif du fond 5 de canal, en amont de la deuxième extrémité 7. Un tel élargissement permet de diminuer la force de capillarité s'appliquant sur le liquide, laquelle constitue le moteur de l'écoulement. Il en résulte une diminution de la vitesse de progression du liquide le long du fond 5 de canal. Lorsque le fond 5 de canal est une ligne, formée par l'intersection des parois latérales 3 et 4, au niveau de l'élargissement, le fond 5 de canal prend la forme d'une surface plane, dont la largeur passe progressivement d'une valeur négligeable (largeur d'une ligne) à 50 $\mu$m, voire entre 150 et 200 $\mu$m.

Les moyens de blocage précédemment décrits peuvent être combinés. En particulier, le dispositif peut comporter un premier moyen de blocage, disposé en amont d'une paroi de fermeture du canal. Une telle combinaison permet de ralentir l'écoulement du liquide en amont de ladite paroi de fermeture.

Le dispositif 1 peut comporter une zone de remplissage de l'échantillon de liquide L située au niveau de la première extrémité 6 du canal 2 et une zone d'arrêt du liquide L située au niveau de la deuxième extrémité 7 du canal 2.

Une zone d'analyse 15, notamment une zone de mesure optique, peut être prévue sur l'étendue du canal 2, notamment dans une partie centrale du canal 2, comme on peut le voir sur la figure 1A. Cette zone d'analyse 15 peut permettre la détermination d'un ou plusieurs paramètres de l'échantillon de liquide L par un moyen d'analyse approprié au niveau de celle-ci, notamment un moyen d'analyse optique apte à diriger un faisceau optique sur cette zone d'analyse 15. De préférence, les parois internes 3 et 4 peuvent ainsi être au moins partiellement transparentes ou translucides, notamment au niveau de la zone d'analyse 15 du canal 2.

Par ailleurs, comme on peut le voir plus facilement sur la figure 1C, le canal 2 se divise en une partie inférieure 11 comportant le fond 5 de canal et une partie supérieure 12 de telle sorte que la partie inférieure 11 se situe entre le fond 5 de canal et la partie supérieure 12. Les parties inférieure 11 et supérieure 12 sont délimitées parles parois internes 3 et 4, la partie inférieure 11 présentant une force capillaire supérieure à celle de la partie supérieure 12 de façon à permettre un écoulement capillaire spontané ECS du liquide L le long du fond 5 de canal depuis la première extrémité 6 vers la deuxième extrémité 7.

La séparation entre les parties inférieure 11 et supérieure 12 du canal 2 peut être réalisée par l'intermédiaire de moyens d'ancrage 13 du liquide L sur au moins une paroi interne 3, 4 du dispositif 1 permettant un blocage de l'écoulement de liquide L depuis la partie inférieure 11 vers la partie supérieure 12.

Plus particulièrement, comme on peut le voir notamment sur la figure 1B, le canal 2 peut comporter deux moyens d'ancrage situés respectivement sur chaque paroi interne 3, 4, ces moyens d'ancrage étant sous la forme d'arêtes d'ancrage 13. De préférence, ces moyens d'ancrage s'étendent de la première extrémité 6 vers la deuxième extrémité 7, parallèlement au fond 5 de canal. Cela permet de constituer une partie inférieure 11 dont la profondeur est constante de la première extrémité 6 vers la deuxième extrémité 7.

Les arêtes d'ancrage 13 peuvent ainsi permettre d'éviter un écoulement du liquide L vers l'extérieur E du dispositif 1, au-delà de la partie inférieure 11 du canal 2.

Autrement dit, grâce aux arêtes d'ancrage 13 et aux arêtes de blocage 9, il est possible de maintenir un confinement du liquide L dans la partie inférieure 11 du canal 2, comme schématisé par la zone en pointillés sur la figure 1A.

L'élargissement du canal 2 obtenu par l'intermédiaire des arêtes de blocage 9 peut dépendre de l'angle $\Omega$ formé par une arête de blocage 9 avec une paroi interne 3 ou 4 du dispositif 1 en cas de non élargissement. Plus spécifiquement, en cas de non élargissement (non présence d'une arête de blocage 9), cet angle $\Omega$ est nul. En cas d'élargissement, comme on peut le voir sur la figure 1B, l'arête de blocage 9 peut par exemple former un angle $\Omega$ supérieur à 20°.

Par ailleurs, l'élargissement obtenu par la présence des arêtes d'ancrage 13 depuis la partie inférieure 11 du canal 2 vers la partie supérieure 12 du canal 2 peut dépendre de l'angle formé par une arête d'ancrage 13 avec une paroi interne 3 ou 4 du dispositif 1 en cas de non élargissement. Plus spécifiquement, en cas de non élargissement (non présence d'une arête d'ancrage 13), cet angle est nul. En cas d'élargissement, l'arête d'ancrage 13 peut former un angle $\delta$ supérieur à 20°, étant notamment égale à 90° sur l'exemple représenté sur la figure 1C.

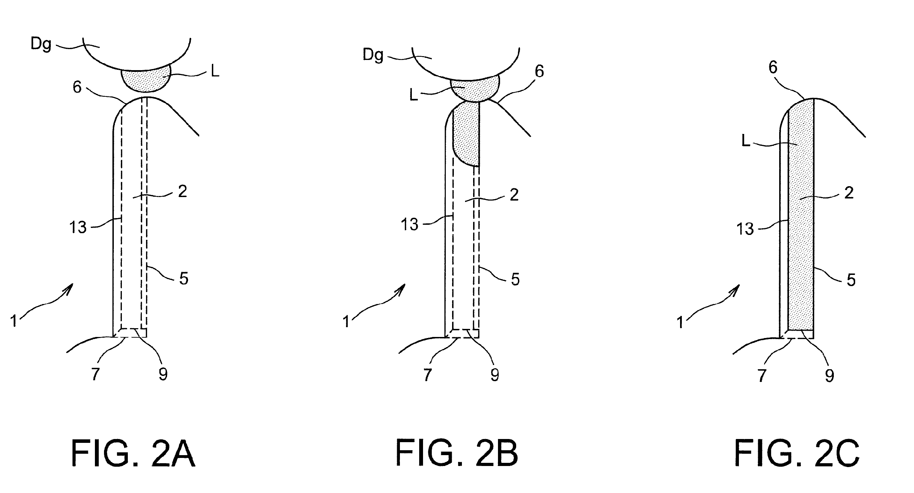

On a représenté sur les figures 2A, 2B et 2C les étapes du prélèvement d'un échantillon de liquide L par le dispositif 1 de prélèvement des figures 1A, 1B et 1C.

Le dispositif 1 peut par exemple être utilisé pour prélever une goutte de sang L formée à l'extrémité d'un doigt $D_g$ d'un utilisateur, destinée à être analysée.

Pour permettre le bon remplissage du dispositif 1 avec le liquide L, le dispositif 1 est placé au contact de la goutte de sang par l'intermédiaire de la première extrémité 6 de collecte du canal 2, comme on peut le voir sur la figure 2A.

Alors, comme on peut le voir sur la figure 2B, le canal 2 se remplit par capillarité en liquide L, le liquide L s'étendant le long du fond 5 de canal depuis la première extrémité 6 en direction de la deuxième extrémité 7.

Puis, comme on peut le voir sur la figure 2C, le liquide L remplit complètement le canal 2 en étant confiné à l'intérieur de la partie inférieure 11 du canal 2 par blocage du liquide L grâce aux arêtes de blocage 9 et aux arêtes d'ancrage 13.

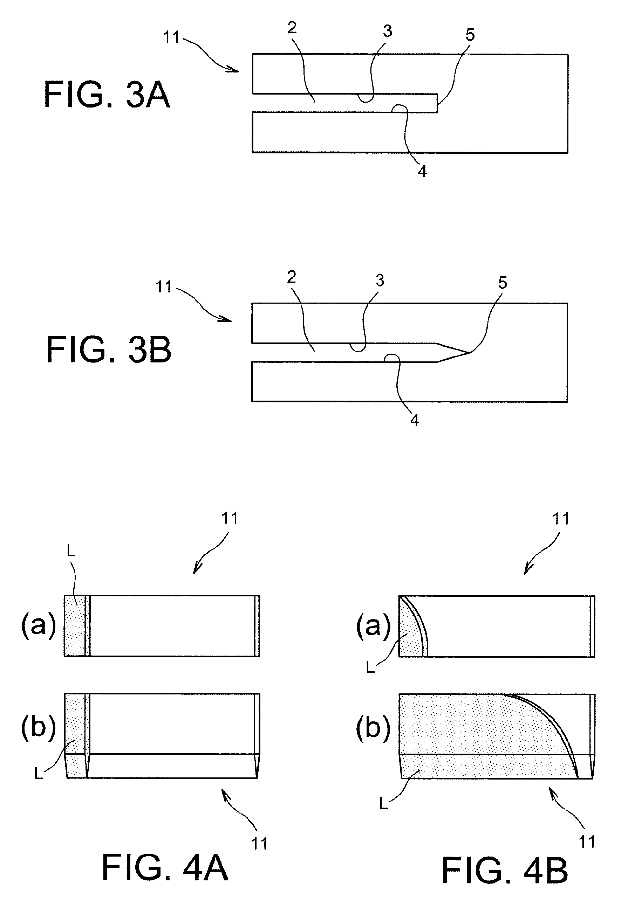

Les figures 3A et 3B représentent deux exemples de réalisation de la partie inférieure 11 du canal 2. Dans l'exemple de la figure 3A, le fond 5 de canal est constitué par une paroi perpendiculaire aux parois internes 3 et 4 du dispositif 1, alors que dans l'exemple de la figure 3B, les parois internes 3 et 4 sont sécantes de sorte à former le fond 5 de canal à leur intersection.

De préférence, le fond 5 de canal est formé par l'intersection de parois internes 3 et 4 sécantes, comme représenté sur la figure 3B.

En effet, les figures 4A et 4B illustrent des résultats de simulation concernant la vitesse de remplissage de parties inférieures 11 de canal 2. Ces simulations ont été réalisées par une méthode d'éléments finis en tenant compte des forces de capillarité pour un angle de mouillage $\theta$ entre le liquide L et une paroi interne inférieur à 78°.

La figure 4A représente une partie inférieure 11 de canal 2 du type de celle de la figure 3A (pour le cas a) et une partie inférieure 11 de canal 2 du type de celle de la figure 3B (pour le cas b) à un instant initial. La figure 4B représente la comparaison entre ces mêmes parties inférieures 11 de canal 2 à un instant t de remplissage.

Ainsi, lorsque les deux parties inférieures 11 sont mises en contact au même instant initial (figure 4A) et lorsque l'on observe le remplissage en liquide L de ces deux parties inférieures 11 à un instant t donné (figure 4B), on constate que la partie inférieure 11 ayant un fond 5 de canal 2 formé par l'intersection de parois internes 3 et 4 sécantes (cas b) se remplit beaucoup plus vite que la partie inférieure 11 de canal 2 ayant un fond 5 de canal formé par une paroi perpendiculaire aux parois internes 3 et 4 (cas a). De plus, la force de capillarité plus importante du fond 5 de canal de forme conique (cas b) permet de créer un remplissage plus rapide tout en limitant la formation de bulles d'air.

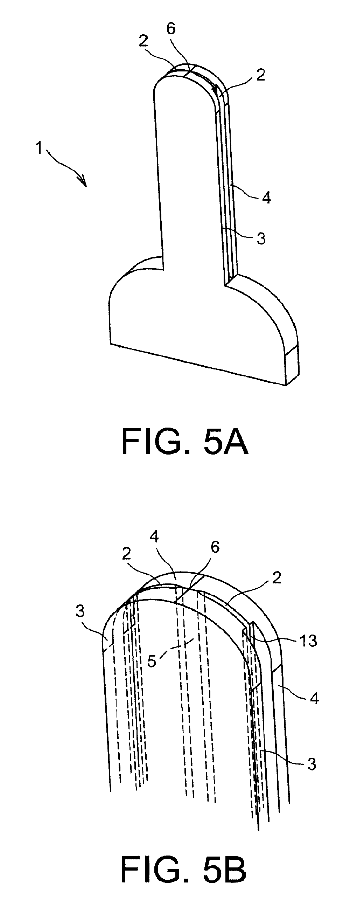

On a représenté sur les figures 5A et 5B un deuxième exemple de réalisation d'un dispositif 1 de prélèvement d'un échantillon de liquide L par capillarité conforme à l'invention. La figure 5A représente un tel dispositif 1 en perspective et la figure 5B est une vue agrandie de ce dispositif 1.

Dans cet exemple, le canal 2 est réalisé de façon semblable à celui de l'exemple des figures 1A, 1B et 1C. Toutefois, dans la partie supérieure 12 du canal 2, les parois internes 3 et 4 sont parallèles entre elles alors que dans l'exemple des figures 1A, 1B et 1C, les parois internes 3 et 4 sont orientées de façon oblique en direction du fond 5 de canal.

Le dispositif 1 des figures 5A et 5B comporte avantageusement deux canaux 2 disposés de façon juxtaposés l'un à l'autre, de sorte que les deux canaux 2 présentent une même première extrémité 6 destinée à entrer en contact avec l'échantillon de liquide L lors du prélèvement.

De façon générale, le dispositif 1 peut comporter dans le canal 2 un réactif, notamment sous forme sèche ou lyophilisée, destiné à réagir avec l'échantillon de liquide L, ce réactif étant notamment situé en amont de la deuxième extrémité 7 en considérant l'écoulement de liquide L depuis la première extrémité 6 vers la deuxième extrémité 7.

Dans le cas du dispositif 1 des figures 5A et 5B, chaque canal 2 peut être rempli d'un même réactif ou par des réactifs différents les uns des autres de façon à pouvoir réaliser des tests de diagnostic multiparamétriques.

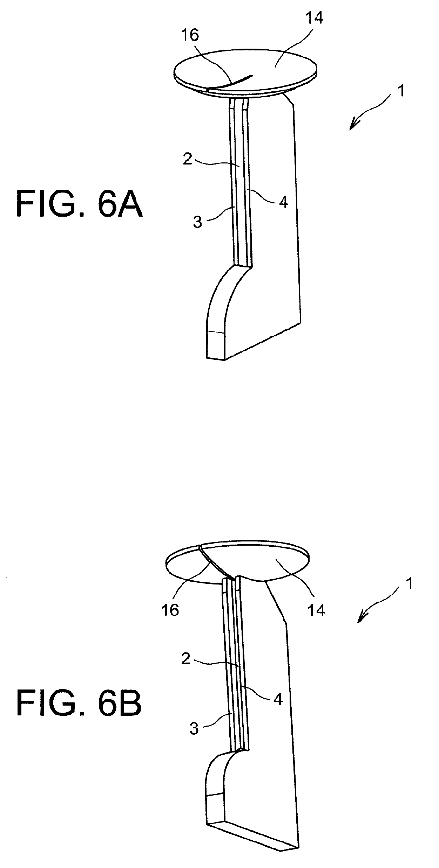

On a représenté sur les figures 6A et 6B un troisième exemple de réalisation d'un dispositif 1 de prélèvement d'un échantillon de liquide L par capillarité conforme à l'invention.

Dans cet exemple, le dispositif 1 est quasiment semblable à celui décrit en référence aux figures 1A, 1B et 1C. Toutefois, le dispositif 1 comporte, au niveau de la première extrémité 6 du canal 2, une surface de contact 14 permettant le prélèvement de l'échantillon de liquide L, cette surface de contact 14 s'étendant dans un plan sensiblement perpendiculaire aux parois internes 3 et 4 du dispositif 1.

La surface de contact 14 comporte en outre une ouverture d'écoulement 16 prévue pour déboucher dans le canal 2.

La surface de contact 14 est avantageusement destinée à être approchée, voire à entrer en contact, avec un élément dont on souhaite prélever un échantillon de liquide L, cet élément pouvant notamment être un élément corporel par exemple un doigt, une lèvre ou tout autre membre dont on souhaite prélever un liquide corporel. De la sorte, la surface de contact 14 peut être configurée de façon à pouvoir être approchée d'un tel élément corporel, voire entrer en contact avec cet élément corporel. En particulier, la surface de contact 14 peut être bombée, étant orientée de façon concave par rapport à l'élément dont on souhaite prélever l'échantillon de liquide L, comme on peut le voir sur les figures 6A et 6B. Cela permet de guider le liquide corporel prélevé vers l'ouverture d'écoulement 16 du canal 2.

La présence d'une telle surface de contact 14 peut permettre d'augmenter le rendement de collecte et également d'éviter que du liquide L prélevé ne s'écoule à l'extérieur E du dispositif 1.

Avantageusement, la surface de contact 14 est configurée pour que sa partie centrale n'entre pas en contact avec l'élément dont on souhaite prélever un échantillon de liquide L. L'ouverture d'écoulement 16 peut être formée au moins en partie dans la partie centrale de la surface de contact 14 de sorte à déboucher au niveau de la partie inférieure 11, notamment au niveau du fond 5 du canal.

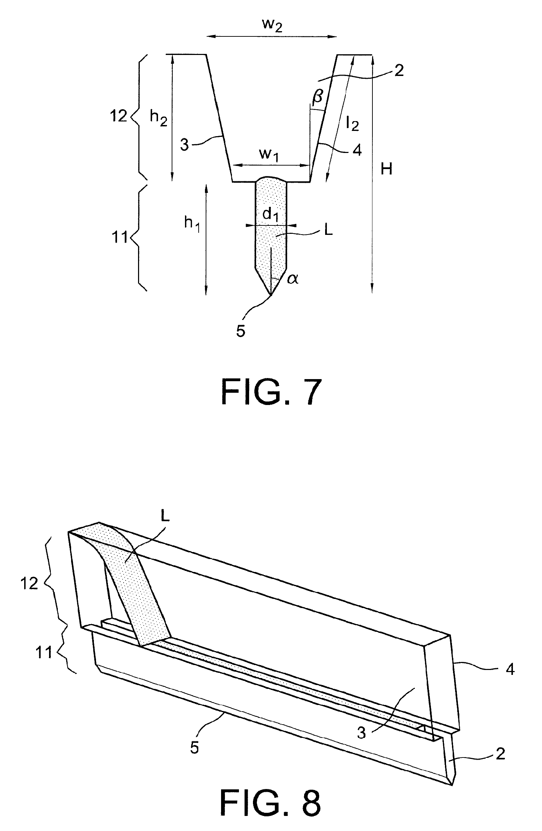

Les figures 7 à 11 servent à illustrer des considérations sur les conditions pour obtenir un écoulement capillaire spontané ECS par capillarité dans le dispositif 1 et permettre également de définir des dimensions du dispositif 1.

La figure 7 représente partiellement un premier exemple de dispositif 1 comportant un canal 2 divisé en une partie inférieure 11 et une partie supérieure 12. Les parois internes 3 et 4 sont sécantes pour former le fond 5 de canal.

Ainsi, le dispositif 1 se présente globalement sous la forme d'un fin capillaire V dans la partie inférieure 11 avec un élargissement dans la partie supérieure 12. La pointe formée par le fond 5 de canal permet de former un filament qui progresse de façon ininterrompu par ce que l'on appelle un effet Conçus-Finn. La partie supérieure 12 est dimensionnée pour ne pas se remplir de liquide L, et peut permettre la manipulation du dispositif 1.

L'écoulement capillaire est facilité par le fait d'avoir un dièdre en V. La condition pour obtenir un écoulement capillaire est la condition de Conçus-Finn, qui s'énonce de la manière suivante :

$$\theta < \frac{\pi}{2} - \alpha,$$

où $\theta$ est l'angle de contact du liquide L avec les parois internes 3 et 4, encore appelé angle de mouillage ou angle de Young,

$\alpha$ est le demi-angle du dièdre, comme représenté sur la figure 7.

Par exemple, si $\alpha$ est égal à 5°, alors il faut que $\theta$ soit strictement inférieur à 85°.

L'effet Conçus-Finn permet d'obtenir un « effet de pointe » lors de l'écoulement du liquide L dans le canal 2, comme on peut le voir sur la figure 8.

La condition exposée ci-dessus dite de Conçus-Finn peut permettre de dimensionner la partie inférieure 11 du canal 2 en fonction de l'angle de contact $\theta$.

Toutefois, il peut être nécessaire en pratique de prendre quelques précautions supplémentaires pour tenir compte des imperfections de surface, et ainsi choisir un angle légèrement plus faible que celui obtenu par la formule théorique de Conçus-Finn.

Il est également nécessaire de pouvoir dimensionner la partie supérieure 12 du canal 2 afin d'obtenir un écoulement capillaire spontané ECS qui permette un écoulement par capillarité le long du fond 5 de canal dans la partie inférieure 11, comme représenté sur la figure 8, mais sans envahir la partie supérieure 12 du canal 2.

Pour ce faire, on suppose que la largeur $d_1$ du canal 2 dans la partie inférieure 11 et que le demi-angle $\alpha$, tels que représentés sur la figure 7, sont connus.

Alors, une étude réalisée avec le logiciel Evolver, a permis de montrer que l'on peut éviter l'envahissement de la partie supérieure 12 du canal 2 par une augmentation appropriée de la section de passage $w_1$ de la partie supérieure 12 du canal 2, comme représenté sur la figure 7.

La condition pour obtenir un écoulement capillaire spontané ECS, déterminée à partir de l'équation thermodynamique de Gibbs, s'écrit alors :

$$w_2 / (2l_2 + (w_1 - d_1)) < \cos \theta.$$

Soit, après la considération géométrique selon laquelle $h_2 = l_2 \times \cos \beta$, la condition pour ne pas avoir d'écoulement capillaire spontané ECS dans la partie supérieure 12 du canal 2 est la suivante :

$$h_2 < [w_1 \cos \beta (1 - \cos \theta) + d_1 \cos \beta \cos \theta] / [2(1-\sin \beta)].$$

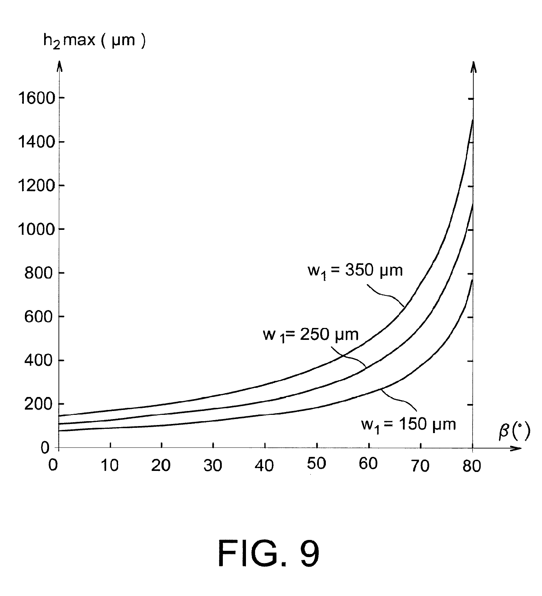

La figure 9 illustre la relation exposée ci-dessus pour le cas où $d_1$ est égale à 100 $\mu$m et $\theta$ est égale à 70°, et ce pour trois valeurs de $w_1$ égales à 150, 250 et 350 $\mu$m. Plus particulièrement, les trois courbes représentées sur la figure 9 représentent la hauteur maximale $h_{2max}$ de la partie supérieure 12 du canal 2 en fonction de l'angle d'inclinaison $\beta$.

Ainsi par exemple, dans le cas d'un angle $\beta$ choisi égal à environ 10°, comme sur l'exemple de la figure 7, il y a intérêt à augmenter $w_1$ afin d'obtenir une hauteur $h_2$ qui ne soit pas trop faible. Par exemple, on peut avoir $h_2$ égale à environ 170 $\mu$m pour $w_1$ choisi égale à environ 350 $\mu$m.

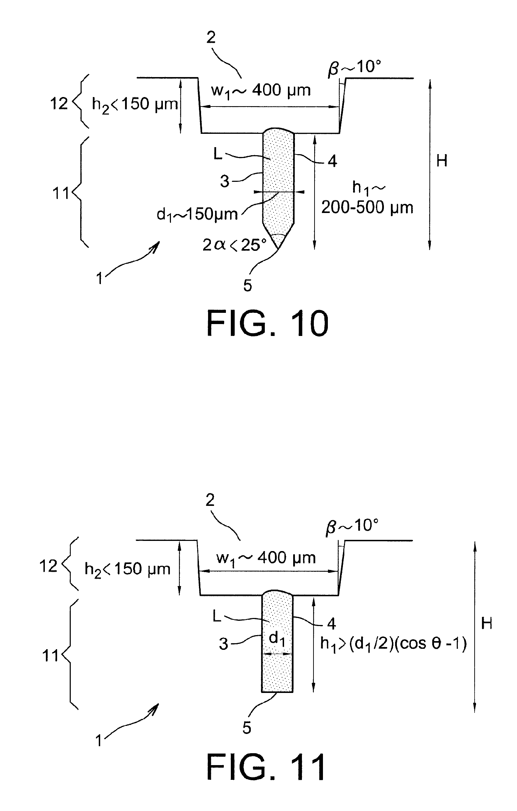

Les figures 10 et 11 représentent deux exemples de canaux 2 de dispositif 1, qui diffèrent en ce que, dans l'exemple de la figure 10, le fond 5 de canal est formé par l'intersection des parois internes 3 et 4 formant une pointe, alors que dans l'exemple de la figure 11, le fond 5 de canal est une paroi s'étendant perpendiculairement entre les parois internes 3 et 4.

Par exemple, dans le cas de la figure 10, un dimensionnement satisfaisant des parties inférieure 11 et supérieure 12 du canal 2 consiste à avoir une pointe assez fine au niveau du fond 5 de canal. La hauteur $h_1$ de la partie inférieure 11 peut par exemple être de l'ordre de 200 à 500 $\mu$m et la largeur $d_1$ peut par exemple être de l'ordre 150 $\mu$m avec un angle de pointe $2\alpha$ par exemple inférieur à 25°.

La hauteur $h_2$ de la partie supérieure 12 peut par exemple être inférieure à 150 $\mu$m, la largeur $w_1$ peut par exemple être de l'ordre de 400 $\mu$m et l'angle $\beta$ peut être de l'ordre de 10°.

Pour pouvoir avoir un écoulement capillaire spontané ECS dans la partie supérieure 12, on a par exemple intérêt à avoir un élargissement brusque avec une valeur $w_1$ environ égale 400 $\mu$m et une hauteur $h_2$ strictement inférieure à 150 $\mu$m.

Dans l'exemple de la figure 11, le dispositif 1 n'utilise pas l'effet Conçus-Finn mais uniquement la condition d'écoulement capillaire spontané ECS. Celle-ci s'écrit alors de la manière suivante :

$$d_1 / (2h_2 + d_1) < \cos \theta$$

Ainsi, pour obtenir un écoulement capillaire spontané ECS, il faut que le rapport entre la hauteur $h_2$ de la partie inférieure 11 du canal 2 et la largeur $d_1$ respecte la condition suivante :

$$(h_2/d_1) > \frac{1}{2} [(1/\cos \theta)-1]$$

Par exemple, pour $d_1$ égale à 150 $\mu$m et $\theta$ égal 85°, $h_1$ est strictement supérieur à 785 $\mu$m.

Par ailleurs, lorsqu'un dépôt de réactif, par exemple sous forme séchée ou lyophilisée, est prévu dans le canal 2, ce réactif étant destiné à réagir avec l'échantillon de liquide L, il est souhaitable que le dépôt du réactif se fasse de la façon la plus homogène possible.

Dans ces conditions, la hauteur H du canal 2, et notamment la hauteur $h_1$ de la partie inférieure 11 du canal 2, est prévue pour rester sensiblement constante en s'étendant le long du fond 5 de canal depuis la première extrémité 6 vers la deuxième extrémité 7 du canal 2.

De plus, en plus d'être constante le long du fond 5 de canal, la hauteur $h_1$ de la partie inférieure 11 du canal 2 doit de préférence être inférieure ou égale à 5 mm, mieux à 2 mm, voire même à 1 mm, voir encore à 0,7 mm.

En outre, il est également souhaitable que le dispositif 1 puisse se remplir avec l'échantillon de liquide L sans pour autant générer une formation de bulles d'air trop importante.

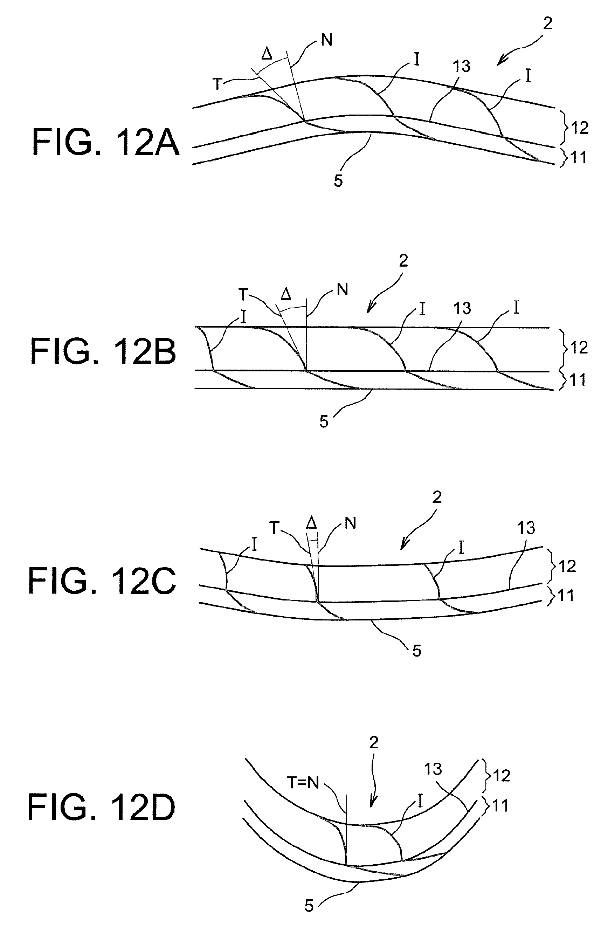

Les figures 12A, 12B, 12C et 12D permettent d'illustrer l'évolution de l'interface I (lignes courbes dans les parties inférieure 11 et supérieure 12 du canal 2 sur les figures 12A à 12D) entre le liquide L et l'air lors du remplissage du dispositif 1 avec l'échantillon de liquide L, et ce pour différentes configurations du canal 2, en particulier pour différentes formes de la génératrice du canal 2 le long du fond 5 de canal.

Ainsi, la figure 12A représente un canal 2 s'étendant selon une direction concave de telle sorte qu'un segment joignant deux points du canal n'est pas nécessairement inclus dans ce canal, la figure 12B représente un canal 2 s'étendant selon une direction plane, et les figures 12C et 12D représentent des canaux 2 s'étendant selon une direction convexe, avec une concavité différente.

Les évolutions des interfaces I entre le liquide et l'air sur les figures 12A à 12D ont été modélisées à l'aide du logiciel Evolver.

La comparaison des figures 12A à 12D montrent qu'au niveau de l'arête d'ancrage 13 marquant la séparation entre les zones inférieure 11 et supérieure 12 du canal 2, la tangente T à l'interface I entre le liquide et l'air forme un angle $\Delta$ avec la normale N à l'arête d'ancrage 13.

Lorsque le canal 2 s'étend selon une direction convexe (cas des figures 12C et 12D), cet angle $\Delta$ est faible, voire nul pour la figure 12D. Ainsi, la tangente T à l'interface I entre le liquide et l'air se rapproche de la normale N à l'arête d'ancrage 13, et cela d'autant plus que la concavité du canal 2 est prononcé.

Dans ces conditions, le risque de formation d'une bulle d'air, notamment au niveau de l'arête d'ancrage 13, est important. En effet, la tangente T à l'interface I, au niveau de l'arête d'ancrage 13, peut se confondre avec la normale N à l'arête d'ancrage 13, voire dépasser cette dernière, et cela en fonction d'hétérogénéités locales, par exemple une fluctuation de l'état de surface. Cette configuration est propice à un remplissage de la partie supérieure 12 plus rapide que le remplissage de la partie inférieure 11 du canal 2, et cela peut ainsi entraîner l'emprisonnement d'une bulle d'air, en particulier au niveau de l'arête d'ancrage 13.

A l'inverse, lorsque le canal 2 s'étend selon une direction rectiligne (cas de la figure 12B) ou concave (cas de la figure 12A), l'angle $\Delta$ est plus élevé, ce qui signifie qu'au niveau de l'arête d'ancrage 13, la tangente T à l'interface I forme un angle élevé, notamment strictement supérieur à 20°, avec la normale N à l'arête d'ancrage 13. Ces conditions sont nettement moins propices à la formation d'une bulle d'air, la vitesse de remplissage de la partie supérieure 12 du canal 2 étant alors similaire à la vitesse de remplissage de la partie inférieure 11 du canal 2.

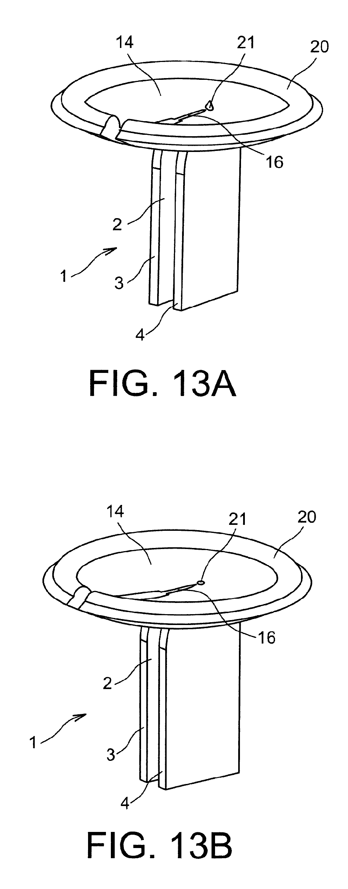

On a représenté sur les figures 13A et 13B un autre exemple de réalisation d'un dispositif 1 de prélèvement d'un échantillon de liquide L par capillarité conforme à l'invention.

Dans cet exemple, le dispositif 1 comporte, comme pour l'exemple de réalisation des figures 6A et 6B, au niveau de la première extrémité 6 du canal 2, une surface de contact 14 permettant le prélèvement de l'échantillon de liquide L, cette surface de contact 14 s'étendant dans un plan sensiblement perpendiculaire aux parois internes 3 et 4 du dispositif 1.

La surface de contact 14 comporte en outre une ouverture d'écoulement 16 prévue pour déboucher dans le canal 2.

La surface de contact 14 est elle-même structurée pour favoriser l'émergence du liquide L corporel issu de l'élément corporel. Ainsi, elle comporte une surface d'appui 20, contre laquelle l'élément corporel est destiné à s'appuyer.

La surface d'appui 20 est annulaire, et centrée par rapport à l'ouverture d'écoulement 16. En particulier, la surface d'appui 20 est, dans cet exemple, constituée par un tore présentant une épaisseur comprise par exemple entre 1 et 5 mm et un diamètre compris par exemple entre 5 mm et 1, 5 cm.

La surface d'appui 16 sous la forme d'un tore circulaire peut permettre de réaliser une surépaisseur pouvant servir d'appui pour l'élément corporel, par exemple un doigt autour de la zone de prélèvement du doigt, c'est-à-dire la zone où une goutte de sang se forme pour le prélèvement, par exemple après action d'un moyen de percement.

Par ailleurs, le dispositif 1 comporte un moyen de percement, notamment sous la forme d'une aiguille 21, pour permette le perçage de la peau afin de collecter l'échantillon de liquide L. Cette aiguille 21 est préférentiellement située à proximité de la première extrémité 6 du canal 2.

L'aiguille 21 peut être mobile dans le dispositif 1 afin de réaliser l'étape d'incision ou de percement de la peau lorsque l'élément corporel est posé en appui sur la surface d'appui 20. Ainsi, l'aiguille 21 mobile dans le dispositif 1 peut être déployée, de façon à être appliquée rapidement contre l'élément corporel, de sorte à former une incision dans ce dernier. Puis, elle peut être repliée dans le dispositif 1. Ainsi, l'aiguille 21 peut être déployée, d'une position de repli, comme illustré sur la figure 13B, vers une position déployée, comme illustré sur la figure 13A, de façon à pratiquer l'incision, puis être repliée dans ladite position de repli.

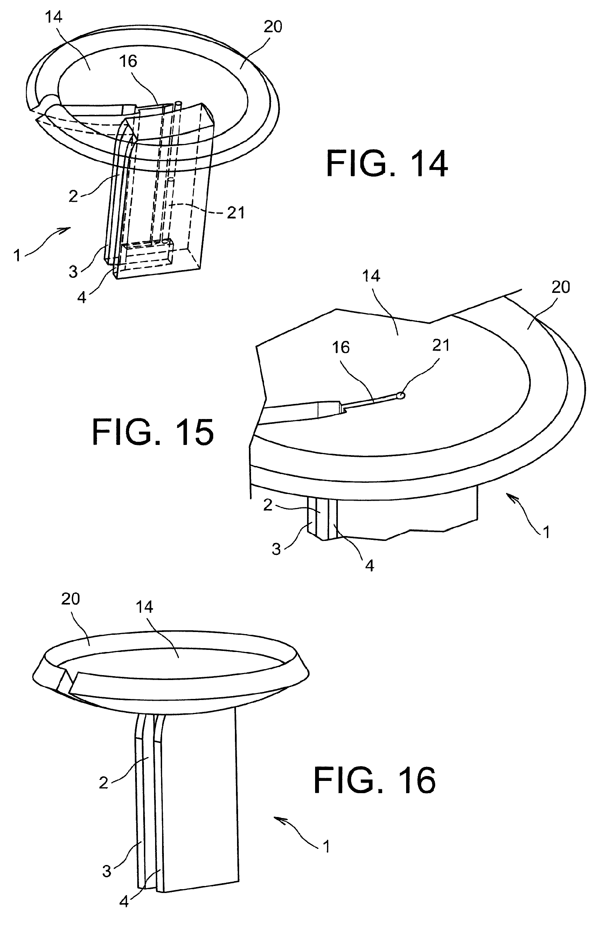

La figure 14 représente la structure interne du dispositif 1 des figures 13A et 13B.

Des moyens de rappel élastiques peuvent par exemple être prévus à l'intérieur du dispositif 1 pour permettre le déplacement du moyen de percement 21 depuis la position de repli (illustrée sur la figure 13B) vers la position déployée (illustrée sur la figure 13A).

Dans une variante non illustrée, l'aiguille 21 peut être fixe. Dans ce cas, le dispositif 1 muni d'une telle aiguille fixe peut d'abord être utilisé dans un instrument de projection permettant à l'aiguille de frapper la peau du patient avec une vitesse pour la projection et une course pour la profondeur de perçage nécessaires pour réaliser une incision suffisante pour faire sortir une goutte d'échantillon de liquide L, par exemple du sang, à la surface de la peau, puis réaliser le retrait de l'aiguille à une vitesse maîtrisée. Puis, le dispositif 1 peut être utilisé pour prélever l'échantillon de liquide L ainsi obtenu, par le biais de la première extrémité 6 du canal 2.

La figure 15 illustre une variante de réalisation du dispositif des figures 13A et 13B. Dans cet exemple, le moyen de percement 21 est situé à une extrémité, notamment une extrémité centrale, de l'ouverture d'écoulement 16 (étant notamment situé dans l'ouverture d'écoulement 16), et non plus à distance de l'ouverture d'écoulement 16, comme représenté sur les figures 13A et 13B. Le positionnement du moyen de percement 21 relativement à l'ouverture d'écoulement 16 peut être choisi de façon à favoriser la collecte du liquide L dans l'ouverture d'écoulement 16 après percement.

La figure 16 représente une variante de réalisation d'un dispositif 1 conforme à l'invention comportant une surface d'appui 20.

Dans cet exemple, la surface d'appui 20 ne se présente plus sous la forme d'un tore circulaire, comme pour les figures 13A et 13B, mais sous la forme d'une arête saillante sur la surface de contact 14, permettant l'appui de l'élément corporel, notamment un doigt, sur le pourtour de la zone de prélèvement de l'élément corporel.

Dans les exemples des figures 13A, 13B et 16, la surface d'appui 20 est circulaire. En variante, la surface d'appui 20 peut également être elliptique. Quoi qu'il en soit, la forme de la surface d'appui 20 peut être choisie de sorte à se conformer à l'élément corporel duquel le liquide corporel L est extrait.

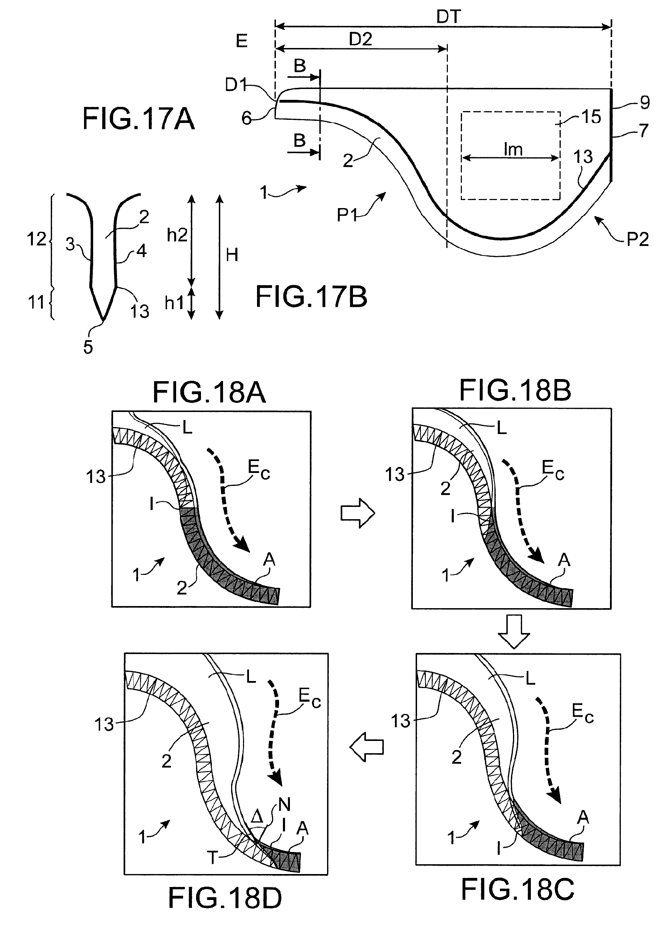

On a par ailleurs représenté, en référence aux figures 17A, 17B et 18A à 18D, un autre exemple de réalisation d'un dispositif 1 de prélèvement conforme à l'invention.

Plus précisément, la figure 17A représente un exemple de réalisation d'un dispositif 1 conforme à l'invention avec une hauteur H du canal 2 qui est variable, la figure 17B est une vue partielle en coupe selon B-B du dispositif 1 de la figure 17A, et les figures 18A à 18D représentent une modélisation de l'écoulement d'un liquide L au cours du temps, respectivement au cours de quatre étapes, dans le canal 2 du dispositif 1 de la figure 17A.

Dans ce mode de réalisation, la hauteur H du canal 2 augmente dans une première portion P1 du canal 2, cette première portion P1 étant située entre une première distance D1 et une deuxième distance D2 par rapport à la première extrémité 6. De plus, la hauteur H du canal 2 diminue dans une deuxième portion P2 du canal 2, cette deuxième portion P2 étant notamment située entre la deuxième distance D2 et la deuxième extrémité 7 du canal 2 où se situe le moyen de blocage 9.

En particulier, dans cet exemple et de manière nullement limitative, la première distance D1 est nulle de sorte que la première portion P1 s'étend de la première extrémité 6 jusqu'à la deuxième distance D2 mesurée à partir de cette première extrémité 6.

Par ailleurs, de façon préférentielle, dans la première portion P1, le canal 2 suit une forme concave de telle sorte qu'un segment joignant deux points du canal 2 n'est pas nécessairement inclus dans ce canal 2. Cette forme concave peut permettre un accroissement rapide de la hauteur H du canal 2.

La première portion P1 peut s'étendre jusqu'à la deuxième extrémité 7.

Dans l'exemple représenté aux figures 17A et 17B, la deuxième distance D2 correspond sensiblement aux deux tiers de la distance DT séparant la première extrémité 6 de la deuxième extrémité 7.

Sur la première portion P1, la hauteur $h_1$ de la partie inférieure 11 du canal 2 est constante, tandis que la hauteur $h_2$ de la partie supérieure 12 du canal 2 augmente progressivement en fonction de la distance par rapport à la première extrémité 6. En particulier, à proximité de la première extrémité 6, le canal 2 s'étend selon une direction concave.

Il a en effet été constaté qu'une telle forme concave, à proximité de la première extrémité 6, permet un meilleur remplissage du canal 2.

Les figures 18A à 18D représentent la modélisation de l'écoulement du liquide L dans le canal 2 du dispositif 1 des figures 17A et 17B, au cours de quatre étapes. Sur ces figures, le sens d'écoulement du liquide L a été représenté par la flèche $E_c$.

On peut ainsi visualiser l'évolution de l'interface I entre le liquide L et l'air A dans le canal 2 du dispositif 1. Ainsi, comme décrit précédemment en référence à la figure 12B, la tangente T à l'interface I, au niveau de l'arête d'ancrage 13, forme un angle $\Delta$ élevé par rapport à la normale N à l'arête d'ancrage 13. Comme expliqué auparavant, cela permet de limiter la formation de bulles d'air.

Par ailleurs, l'accroissement de la hauteur H du canal 2 tel que décrit précédemment peut avantageusement permettre de ménager une zone de mesure optique 15 de grande dimension, par exemple d'une largeur $l_m$ de quelques millimètres, par exemple comprise entre 3 et 4 mm. Cette zone de mesure optique 15 est par exemple située entre la première portion P1 et la deuxième extrémité 7, comme représenté sur la figure 17A.

Bien entendu, l'invention n'est pas limitée aux exemples de réalisation qui viennent d'être décrits. Diverses modifications peuvent y être apportées par l'homme du métier.

L'expression « comportant un » doit être comprise comme étant synonyme de « comportant au moins un », sauf si le contraire est spécifié.

**Revendications**

1. Dispositif (1) de prélèvement d'un échantillon de liquide (L) par capillarité, comportant un canal (2) d'écoulement du liquide délimité par deux parois internes (3, 4) du dispositif (1) entre lesquelles s'étend un fond (5) de canal, la distance (D) séparant les deux parois internes (3, 4) diminuant en direction du fond (5) de canal, le canal (2) s'étendant entre une première extrémité (6) de collecte, ouverte sur l'extérieur (E) du dispositif (1) et apte à recevoir le liquide (L), et une deuxième extrémité (7), de façon à permettre l'écoulement du liquide (L) par capillarité le long du fond (5) de canal depuis la première extrémité (6) vers la deuxième extrémité (7), le canal (2) comportant, au niveau de la deuxième extrémité (7), un moyen de blocage (9, 17) apte à bloquer l'écoulement de liquide (L) dans le canal (2) depuis la première extrémité (6) vers la deuxième extrémité (7).

2. Dispositif selon la revendication 1, **caractérisé en ce que** le canal (2) comporte un moyen de blocage sous la forme d'une paroi de fermeture (17) du canal (2).

3. Dispositif selon la revendication 1 ou 2, **caractérisé en ce que** le canal (2) comporte un moyen de blocage sous la forme d'au moins une arête de blocage (9) formée sur au moins une paroi interne (3, 4) du dispositif (1) permettant un élargissement d'au moins une partie du canal (2).

4. Dispositif selon l'une des revendications 1 à 3, **caractérisé en ce que** le canal (2) comporte un moyen de blocage sous la forme d'un revêtement rendu localement hydrophobe pour empêcher l'écoulement du liquide (L).

5. Dispositif selon l'une des revendications précédentes, **caractérisé en ce que** le canal (2) comporte un moyen de blocage sous la forme d'un élargissement, notamment progressif, du fond (5) de canal, apte à diminuer la force capillaire s'appliquant sur le liquide (L), lorsque ce dernier progresse vers la deuxième extrémité (7).

6. Dispositif selon l'une quelconque des revendications précédentes, **caractérisé en ce que** le canal (2) se divise en au moins une partie inférieure (11) comportant le fond (5) de canal et une partie supérieure (12) de telle sorte que la partie inférieure (11) se situe entre le fond (5) de canal et la partie supérieure (12), les parties inférieure (11) et supérieure (12) étant délimitées par les parois internes (3, 4), la partie inférieure (11) présentant une force capillaire supérieure à celle de la partie supérieure (12) de façon à permettre un écoulement capillaire spontané du liquide (L) le long du fond (5) de canal depuis la première extrémité (6) vers la deuxième extrémité (7).

7. Dispositif selon l'une quelconque des revendications précédentes, **caractérisé en ce que** le canal (2) comporte sur au moins une partie de celui-ci au moins un réactif sous forme sèche et/ou lyophilisée.

8. Dispositif selon l'une quelconque des revendications précédentes, **caractérisé en ce que** le canal (2) s'étend selon une direction sensiblement rectiligne depuis la première extrémité (6) vers la deuxième extrémité (7).

9. Dispositif selon l'une quelconque des revendications 1 à 7, **caractérisé en ce que** le canal (2) s'étend selon une direction sensiblement concave depuis la première extrémité (6) vers la deuxième extrémité (7).

10. Dispositif selon l'une quelconque des revendications précédentes, **caractérisé en ce que** le dispositif (1) comporte, au niveau de la première extrémité (6) du canal (2), une surface de contact (14) permettant le prélèvement de l'échantillon de liquide (L) s'étendant dans un plan sensiblement perpendiculaire à au moins une paroi interne (3, 4) du dispositif (1).

11. Dispositif selon la revendication 10, **caractérisé en ce que** la surface de contact (14) comporte une ouverture d'écoulement (16) prévue pour déboucher dans le canal (2).

**12.** Dispositif selon l'une quelconque des revendications précédentes, **caractérisé en ce que** le dispositif (1) comporte un moyen de percement (21), notamment une aiguille, pour permettre le perçage de la peau afin de collecter l'échantillon de liquide (L).

**13.** Dispositif selon l'une quelconque des revendications précédentes, **caractérisé en ce que** la hauteur (H) du canal (2) est variable, augmentant au moins sur une première portion (P1) du canal (2).

**14.** Dispositif selon la revendication 13, **caractérisé en ce que** le canal (2) comporte une partie concave, s'étendant sur une première portion (P1) comprise entre une première distance (D1) et une deuxième distance (D2) par rapport à la première extrémité (6) du canal (2).

**15.** Procédé d'analyse d'un échantillon de liquide (L) prélevé au moyen d'un dispositif (1) selon l'une quelconque des revendications précédentes, dans lequel on soumet le dispositif (1) à des moyens d'analyse, aptes à analyser le liquide (L) contenu dans le canal (2) au niveau d'au moins une zone d'analyse (15) prédéterminée du canal (2).

**Patentansprüche**

**1.** Vorrichtung (1) zur Entnahme einer Probe von Flüssigkeit (L) durch Kapillareffekt, umfassend einen Strömungskanal (2) für die Flüssigkeit, der zwischen zwei Innenwänden (3, 4) der Vorrichtung (1) begrenzt ist, zwischen denen sich ein Boden (5) des Kanals erstreckt, wobei der Abstand (D), der die zwei Innenwände (3, 4) trennt, in Richtung des Bodens (5) des Kanals abnimmt, wobei sich der Kanal (2) zwischen einem ersten Sammelende (6), das zur Außenseite (E) der Vorrichtung (1) hin offen und dazu ausgelegt ist, die Flüssigkeit (L) aufzunehmen, und einem zweiten Ende (7) derart erstreckt, dass das Strömen der Flüssigkeit (L) durch Kapillareffekt entlang des Bodens (5) des Kanals vom ersten Ende (6) zum zweiten Ende (7) ermöglicht ist, wobei der Kanal (2) im Bereich des zweiten Endes (7) ein Blockiermittel (9, 17) umfasst, das dazu ausgelegt ist, das Strömen von Flüssigkeit (L) in dem Kanal (2) vom ersten Ende (6) zum zweiten Ende (7) zu blockieren.

**2.** Vorrichtung nach Anspruch 1, **dadurch gekennzeichnet, dass** der Kanal (2) ein Blockiermittel in Form einer Wand (17) zum Schließen des Kanals (2) umfasst.

**3.** Vorrichtung nach Anspruch 1 oder 2, **dadurch gekennzeichnet, dass** der Kanal (2) ein Blockiermittel in Form einer Blockierkante (9) umfasst, die an wenigstens einer Innenwand (3, 4) der Vorrichtung (1) zum Ermöglichen einer Aufweitung wenigstens eines Teils des Kanals (2) gebildet ist.

**4.** Vorrichtung nach einem der Ansprüche 1 bis 3, **dadurch gekennzeichnet, dass** der Kanal (2) ein Blockiermittel in Form einer Beschichtung umfasst, die lokal hydrophob gemacht ist, um das Strömen der Flüssigkeit (L) zu verhindern.

**5.** Vorrichtung nach einem der vorhergehenden Ansprüche, **dadurch gekennzeichnet, dass** der Kanal (2) ein Blockiermittel in Form einer insbesondere progressiven Aufweitung des Bodens (5) des Kanals umfasst, die dazu ausgelegt ist, die auf die Flüssigkeit (L) ausgeübte Kapillarkraft zu verringern, wenn sich letztere zum zweiten Ende (7) hin vorwärts bewegt.

**6.** Vorrichtung nach einem der vorhergehenden Ansprüche, **dadurch gekennzeichnet, dass** sich der Kanal (2) wenigstens in einen unteren Teil (11), der den Boden (5) des Kanals umfasst, und einen oberen Teil (12) derart aufteilt, dass sich der untere Teil (11) zwischen dem Boden (5) des Kanals und dem oberen Teil (12) befindet, wobei der untere (11) und der obere (12) Teil durch die Innenwände (3, 4) begrenzt sind, wobei der untere Teil (11) eine Kapillarkraft aufweist, die größer ist als jene des oberen Teils (12), derart, dass eine spontane Kapillarströmung der Flüssigkeit (L) entlang des Bodens (5) des Kanals vom ersten Ende (6) zum zweiten Ende (7) ermöglicht ist.

**7.** Vorrichtung nach einem der vorhergehenden Ansprüche, **dadurch gekennzeichnet, dass** der Kanal (2) wenigstens auf einem Teil desselben wenigstens ein Reagenz in trockener und/oder lyophilisierter Form umfasst.

**8.** Vorrichtung nach einem der vorhergehenden Ansprüche, **dadurch gekennzeichnet, dass** sich der Kanal (2) entlang einer im Wesentlichen geradlinigen Richtung vom ersten Ende (6) zum zweiten Ende (7) erstreckt.

**9.** Vorrichtung nach einem der Ansprüche 1 bis 7, **dadurch gekennzeichnet, dass** sich der Kanal (2) entlang einer im Wesentlichen konkaven Richtung vom ersten Ende (6) zum zweiten Ende (7) erstreckt.

10. Vorrichtung nach einem der vorhergehenden Ansprüche, **dadurch gekennzeichnet, dass** die Vorrichtung (1) im Bereich des ersten Endes (6) des Kanals (2) eine Kontaktoberfläche (14) umfasst, die die Entnahme der Probe von Flüssigkeit (L) ermöglicht, die sich in einer Ebene im Wesentlichen orthogonal zu wenigstens einer Innenwand (3, 4) der Vorrichtung (1) erstreckt.

11. Vorrichtung nach Anspruch 10, **dadurch gekennzeichnet, dass** die Kontaktoberfläche (14) eine Strömungsöffnung (16) umfasst, die dazu ausgelegt ist, in den Kanal (2) zu münden.

12. Vorrichtung nach einem der vorhergehenden Ansprüche, **dadurch gekennzeichnet, dass** die Vorrichtung (1) ein Durchstichmittel (21), insbesondere eine Nadel, umfasst, um das Durchstoßen der Haut zum Zweck des Sammelns der Probe von Flüssigkeit (L) zu ermöglichen.

13. Vorrichtung nach einem der vorhergehenden Ansprüche, **dadurch gekennzeichnet, dass** die Höhe (H) des Kanals (2) variabel ist und wenigstens über einen ersten Bereich (P1) des Kanals (2) zunimmt.

14. Vorrichtung nach Anspruch 13, **dadurch gekennzeichnet, dass** der Kanal (2) einen konkaven Teil umfasst, der sich über einen ersten Bereich (P1) erstreckt, der zwischen einem ersten Abstand (D1) und einem zweiten Abstand (D2) bezogen auf das erste Ende (6) des Kanals (2) enthalten ist.

15. Verfahren zur Analyse einer Probe von Flüssigkeit (L), die mittels einer Vorrichtung (1) nach einem der vorhergehenden Ansprüche entnommen ist, wobei man die Vorrichtung (1) Analysemitteln unterzieht, die dazu ausgelegt sind, die in dem Kanal (2) enthaltene Flüssigkeit (L) im Bereich wenigstens einer vorbestimmten Analysezone (15) des Kanals (2) zu analysieren.

**Claims**

1. Device (1) for taking a sample of liquid (L) by capillarity, comprising a channel (2) for flow of the liquid delimited by two internal walls (3, 4) of the device (1) between which a channel bottom (5) extends, the distance (D) separating the two internal walls (3, 4) decreasing in the direction of the channel bottom (5), the channel (2) extending between a first collecting end (6), open onto the outside (E) of the device (1) and able to receive the liquid (L), and a second end (7), so as to enable the liquid (L) to flow by capillarity along the channel bottom (5) from the first end (6) towards the second end (7), the channel (2) comprising, at the second end (7), a blocking means (9, 17) able to block the flow of liquid (L) in the channel (2) from the first end (6) towards the second end (7).

2. Device according to claim 1, **characterised in that** the channel (2) comprises a blocking means in the form of a closure wall (17) of the channel (2).

3. Device according to claim 1 or 2, **characterised in that** the channel (2) comprises a blocking means in the form of at least one blocking ridge (9) formed on at least one internal wall (3, 4) of the device (1), allowing a broadening of at least part of the channel (2).

4. Device according to one of claims 1 to 3, **characterised in that** the channel (2) comprises a blocking means in the form of a coating made locally hydrophobic in order to prevent the flow of liquid (L).

5. Device according to one of the preceding claims, **characterised in that** the channel (2) comprises a blocking means in the form of a broadening, in particular gradual, of the channel bottom (5), able to reduce the capillary force applied to the liquid (L), when the latter progresses towards the second end (7).

6. Device according to any one of the preceding claims, **characterised in that** the channel (2) is divided into at least a lower part (11) comprising a channel bottom (5) and an upper part (12) so that the lower part (11) is situated between the channel bottom (5) and the upper part (12), the lower (11) and upper (12) parts being delimited by the internal walls (3, 4), the lower part (11) having a capillary force greater than that of the upper part (12) so as to allow a spontaneous capillary flow of the liquid (L) along the channel bottom (5) from the first end (6) towards the second end (7).

7. Device according to any one of the preceding claims, **characterised in that** the channel (2) comprises, on at least part thereof, at least one reagent in dry and/or lyophilised form.

8. Device according to any one of the preceding claims, **characterised in that** the channel (2) extends in a substantially rectilinear direction from the first end (6) towards the second end (7).

9. Device according to any one of claims 1 to 7, **characterised in that** the channel (2) extends in a substantially concave direction from the first end (6) towards the second end (7).

10. Device according to any one of the preceding claims, **characterised in that** the device (1) comprises, at the first end (6) of the channel (2), a contact surface (14) enabling the liquid sample (L) to be taken, lying in a plane substantially perpendicular to at least one internal wall (3, 4) of the device (1).

11. Device according to claim 10, **characterised in that** the contact surface (14) comprises a flow opening (16) designed to emerge in the channel (2).

12. Device according to any one of the preceding claims, **characterised in that** the device (1) comprises a piercing means (21), in particular a needle, to enable the skin to be pierced so as to collect the liquid sample (L).

13. Device according to any one of the preceding claims, **characterised in that** the height (H) of the channel (2) is variable, increasing at least over a first portion (P1) of the channel (2).

14. Device according to claim 13, **characterised in that** the channel (2) comprises a concave part, extending over a first portion (P1) lying between a first distance (D1) and a second distance (D2) with respect to the first end (6) of the channel (2).

15. Method for analysing a liquid sample (L) taken by means of a device (1) according to any one of the preceding claims, in which the device (1) is subjected to analysis means able to analyse the liquid (L) contained in the channel (2) at at least one predetermined analysis zone (15) of the channel (2).

FIG. 1A

FIG. 1B

FIG. 1C

FIG. 2A                    FIG. 2B                    FIG. 2C

FIG. 3A

FIG. 3B

(a)

(b)

FIG. 4A

(a)

(b)

FIG. 4B

# FIG. 5A

# FIG. 5B

FIG. 6A

FIG. 6B

# FIG. 7

# FIG. 8

FIG. 9

**FIG. 10**

**FIG. 11**

FIG. 12A

FIG. 12B

FIG. 12C

FIG. 12D

# FIG. 13A

# FIG. 13B

FIG. 14

FIG. 15

FIG. 16

FIG.17A

FIG.17B

FIG.18A

FIG.18B

FIG.18D

FIG.18C

**EP 2 964 090 B1**

## RÉFÉRENCES CITÉES DANS LA DESCRIPTION

**Documents brevets cités dans la description**

- EP 0821784 B1